# EUROPEAN PATENT APPLICATION

(11) **EP 0 773 289 A2**
(43) Date of publication of application: **14.05.1997**
(21) Application number: 96113349.3
(22) Date of filing: 20.08.1996
(51) Int. Cl.: C12N 15/12, C07K 14/705, G01N 33/53

(54) **Asialoglycoprotein receptor derivatives and their use**

(30) Priority: 21.08.1995 JP 212118/95
(71) Applicant: Tonen Corporation, Tokyo (JP)
(72) Inventor: Tanida, Emiko, c/o Tonen Corp., Ooi-machi, Iruma-gun, Saitama (JP); Ohue, Chiharu, c/o Tonen Corp., Ooi-machi, Iruma-gun, Saitama (JP); Yagi, Shintaro, c/o Tonen Corp., Ooi-machi, Iruma-gun, Saitama (JP); Hasegawa, Akira, c/o Tonen Corp., Ooi-machi, Iruma-gun, Saitama (JP); Kiyosawa, Kendo, Matsumoto-shi, Nagano (JP); Yano, Akihiko, Nagasaki University, Nagasaki-shi, Nagasaki (JP)
(74) Representative: Klunker . Schmitt-Nilson . Hirsch

(57) **Abstract**

Derivatives comprising the AGPR H1 and L-H2 extracellular domains and derivatives comprising the AGPR H1 and L-H2 cytoplasmic and extracellular domains; derivatives thereof; a method of producing AGPR H1 and AGPR L-H2; a method of assaying anti-AGPR antibodies using the derivatives; and reagents and a kit to be used for the method.

According to the invention, stable quality reagents may be obtained and accurate measurement may be achieved.

## Description

### BACKGROUND OF INVENTION

### 1. Field of Invention

The present invention relates to derivatives of asialoglycoprotein receptors H1 and L-H2 (AGPR H1 and AGPR L-H2), to a process for production of the AGPR derivatives or AGPR, and to the use thereof. The AGPR derivatives of the invention are useful for the detection of anti-AGPR antibodies, particularly for clinical examinations.

### 2. Related Art

The autoimmune hepatitis (AIH) is an active chronic hepatitis, and often progresses to cirrhosis and even to fatal intractable hepatitis if not treated (with immunosuppressants or the like). Currently diagnosis is made by comparison tests against criteria established by the Health and Welfare Ministry (hypergammaglobulin plasma, anti-nuclear antibody, etc.), but no markers have existed which can be used for definite diagnosis. Furthermore, since a good number of patients who do not match the diagnostic criteria for AIH are still judged to have AIH, it is clear that the present diagnostic criteria are insufficient, and thus the establishment of markers has been desired as a more certain and appropriate measure for the definite diagnosis of autoimmune hepatitis as well.

The difficulty of diagnosing autoimmune hepatitis has been amply documented. For example, the following documents have pointed out that since patients today are diagnosed with hepatitis C, many patients previously diagnosed with autoimmune hepatitis could have been classified as hepatitis C (Lenzi et al., Lancet 335: 258-259, 1990; Lunel et al., Hepatology 16: 630-636, 1992; Cassani, et al. Gut 33: 1260-1263, 1992; Pawlotsky et al. Gut: S66-S68, 1993). Since no simple method has existed as a means of judging whether hepatitis C is active or not (whether the hepatitis C virus is proliferating or whether it is a past HCV infection which is healing), and no accurate diagnostic method for autoimmune hepatitis has been established, it has been difficult to accurately diagnose such patients.

Nevertheless, distinction of the two conditions is highly significant from a clinical viewpoint. That is, while the effect of immunosuppressants (steroids, etc.) on AIH is known, if patients exhibiting AIH-like symptom are instead infected with the hepatitis C virus, administration of immunosuppressants will accelerate growth of the hepatitis C virus, having the opposite effect from healing. On the other hand, interferon which is used for HCV therapy enhances the strength of the immune response, and therefore if interferon is administered to an AIH patient who happens to be HCV antibody-positive, the AIH symptoms will deteriorate, having the opposite effect from healing. Distinction of the two conditions, therefore, has become an important issue for current clinical medicine.

An autoimmune disease is a symptom which occurs when an antigen which should normally be recognized as self is recognized as nonself, and thus the self is attacked by the immune system which is supposed to attack foreign bodies. In the case of autoimmune diseases, the detection of antibodies against the antigen recognized as nonself is an effective diagnostic criterion.

In the case of AIH, anti-nuclear autoantibodies and anti-smooth muscle autoantibodies are detected in such conditions as lupoid hepatitis which are classified as Type I, while anti-Liver-Kidney microsomal type 1 autoantibodies and anti-liver cytosol type 1 autoantibodies, etc. are detected in Type II, and this detection has become one of the criteria of diagnosis. However, detection of these antibodies is also observed in other autoimmune diseases, and no organ specificity has been found. Consequently, definitive diagnosis of autoimmune hepatitis cannot be made based on detection of these antibodies alone, and thus a comprehensive diagnosis is made taking into consideration other symptoms or liver biopsy (invasion of lymph follicles in the liver, etc.). Patients still undergo stress despite progress in, for example, liver biopsy techniques, and therefore it would be preferable to use a simpler method such as blood serum examination. In the light of such circumstances, establishment of an AIH-specific autoantibody detection system has been a desired goal.

Asialoglycoprotein receptor (hereunder abbreviated to AGPR) is a membrane protein expressed specifically by hepatocytes, and it functions to uptake asialoglycoproteins in the serum for degradation in the liver. Reduction in expression of AGPR has been reported in response to such liver conditions as hepatic cirrhosis, liver cancer and regenerated liver (Stadalnik et al., J. Nucl. Med. 26:1233-1242, 1985). It has also be reported that AGPR itself is present in part of the serum (Katsugi et al., Alcohol Metabolism and Liver: Vol.12, pp.65-68, 1992), and significant research is progressing toward the measurement of serum AGPR. Other published documents teach that labelling compounds binding to AGPR can be used as good indicators of liver function (Kudo, et al., Japan Assoc. of Gastrointest. Pathology: Vol.89, pp.1349-1359, 1992).

McFarlane et al. have reported that antibodies which react with AGPR purified from rabbit liver are detected at a high level in active AIH patients, suggesting the possibility that AGPR is a target of AIH (McFarlane et al., J. Hepatol. 3:196-205, 1986). These results have been confirmed by Treichel et al. In other words, the fact that antibodies which react with AGPR purified from human liver are found at high levels in AIH patients and primary biliary cirrhosis patients supports the idea that the appearance of autoantibodies against AGPR can be used as an indicator for AIH (Treichel et al., Hepatol. 11:606-612, 1990). This has been given further support by multicenter research in which the present inventors have also participated (Treichel et al., Gastroenterology 107:799-804, 1994). It has also been demonstrated that production of the antibody is T cell dependent (Loehr et al., Hepatol. 12:1314-1320, 1990).

As described in the aforementioned document, Treichel et al. conducted a comparative study of the reactivities of AGPR purified from rabbits and rats and AGPR purified from human liver, with AIH patient serum, and reported a difference in the reactivities of the human protein and the protein purified from other animal species. That is, human AGPR must be used to measure antibodies against AGPR.

As mentioned above, AGPR is reduced in response to such liver conditions as hepatic cirrhosis, liver cancer and regenerated liver (Stadalnik et al., J. Nucl. Med. 26:1233-1242, 1985), and therefore healthy liver must be used to obtain AGPR. However, since healthy human liver is not easily acquirable, the establishment of a method of preparing AGPR easily and in large amounts has been a desired goal. With purification from liver, there also exists the risk of contamination by liver antigens that would react no specifically in AIH patient, and thus when the proteins purified from liver are used as antigens for antigen/antibody reaction to detect anti-AGPR antibodies, there is the risk that antibodies other than those against AGPR will be detected.

Since AGPR expression has been reported in certain cell lines established from hepatic parenchymal cells, among which are HepG2 cells and HuH7 cells, their use as materials for preparing AGPR is conceivable, but the types of AGPR mainly expressed by HepG2 and normal liver are different (Paietta et al., Hepatol. 15:359-402, 1992), by which it is speculated that the protein prepared from these established cell lines cannot be used in place of that prepared from normal liver. Moreover, as mentioned above, protein from other animal species cannot be substituted for human protein.

### SUMMARY OF THE INVENTION

Consequently, it is an object of the present invention to provide a method for mass production of AGPR of the type expressed by normal human liver and AGPR polypeptide derivatives. It is a further object to provide a method which employs the AGPR to detect anti-AGPR autoantibodies to be used for specific antigen/antibody reactions, and a reagent and kit for diagnosis of AIH by detection of the autoantibodies.

The present invention provides, therefore, asialoglycoprotein receptor (AGPR) derivatives (AGPR derivatives), i.e. AGPR H1 derivative comprising the extracellular domain of asialoglycoprotein receptor H1 (AGPR H1); AGPR L-H2 derivative comprising the extracellular domain of asialoglycoprotein receptor L-H2 (AGPR L-H2); AGPR H1 derivative comprising both the cytoplasmic domain and the extracellular domain of AGPR H1 (without the membrane spanning domain); and AGPR L-H2 derivative comprising the cytoplasmic domain and extracellular domain of AGPR (without the membrane spanning domain). In the present specification, the aforementioned derivatives will sometimes be referred to simply as AGPR, AGPR H1, AGPR L-H2, etc. without the term "derivative".

The present invention further provides a method of producing the above-mentioned AGPR derivatives which is characterized by culturing host cells transformed by an expression vector comprising DNA encoding the polypeptide of the derivatives, and collecting the cultured product or further collecting the AGPR derivatives from the cultured product.

The present invention further provides a method of producing AGPR H1 or AGPR L-H2 which is characterized by culturing host cells transformed by an expression vector comprising DNA encoding the polypeptide amino acid sequence of the AGPR, and collecting the cultured product or further collecting the AGPR from the cultured product.

The present invention still further provides a method of simultaneously producing AGPR H1 and AGPR L-H2 which is characterized by culturing host cells transformed by an expression vector comprising DNA encoding the polypeptide amino acid sequence of AGPR H1 and DNA coding for the polypeptide amino acid sequence of AGPR L-H2, and collecting the cultured product or further collecting the AGPR H1 and AGPR L-H2 from the cultured product.

The present invention still further provides a method of detecting or measuring antibodies against AGPR (anti-AGPR antibody) which is characterized by contacting a test sample to be measured for the presence or amount of the anti-AGPR antibody with at least one type of the above-mentioned AGPR derivatives or the AGPR produced by the above-mentioned methods, and detecting whether an antigen/antibody reaction occurs or measuring its amount

The present invention still further provides a reagent for measuring anti-AGPR antibodies which comprises at least one type of the above-mentioned AGPR derivatives or AGPR obtained by the above-mentioned methods.

The present invention still further provides a kit for measuring anti-AGPR antibodies which comprises at least one type of the above-mentioned AGPR derivatives or AGPR obtained by the above-mentioned methods, and the anti-AGPR antibody as a standard.

### BRIEF DESCRIPTION OF THE DRAWINGS

Fig. 1 is a schematic drawing of the primary structures of AGPR H1 (A) and AGPR L-H2 (B).

Fig. 2 is a graph showing the results of ELISA measurement of each specimen, using a membrane spanning domain-removed AGPR H1 derivative according to the invention as the antigen.

Fig. 3 is a graph showing the results of ELISA measurement of each specimen, using a membrane spanning domain-removed AGPR L-H2 derivative according to the invention as the antigen.

### DETAILED DESCRIPTION

AGPR expressed by normal liver is H1, H2 and L-H2. On the other hand, HepG2 cells express H1, and H2 and H2' which are believed to be a result of alternative splicing from the same gene as L-H2 (Paietta et al., Hepatol. 15:359-402, 1992). Thus, since the protein to be produced according to the invention reflects AGPR expressed in normal liver, the objects are H1 and L-H2 derivatives.

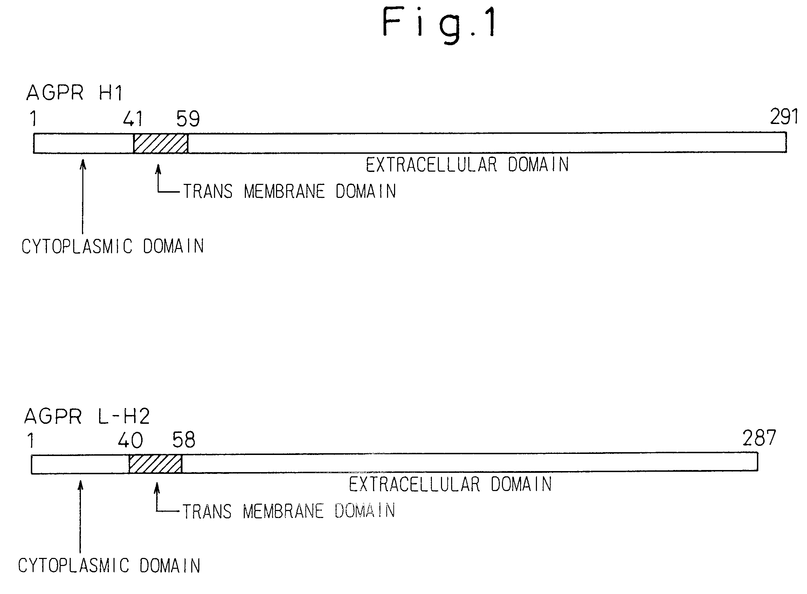

The primary structures of AGPR H1 and AGPR L-H2 are shown in Fig. 1. One of the AGPR derivatives of the invention approximately comprises the extracellular domain of AGPR H1. A specific example thereof is a derivative having the amino acid sequence from the 60th amino acid Gly to the 291st amino acid Leu of the amino acid sequence represented by SEQ ID NO: 19. Another derivative according to the invention approximately comprises the cytoplasmic domain and extracellular domain of AGPR H1. A specific example thereof comprises the cytoplasmic domain having the amino acid sequence from the 1st amino acid Met to the 42nd amino acid Leu, and the extracellular domain having the amino acid sequence from the 60th amino acid Gly to the 291st amino acid Leu, of the amino acid sequence represented by SEQ ID NO: 19.

Another AGPR derivative according to the invention approximately comprises the extracellular domain of AGPR L-H2. A specific example thereof is one having the amino acid sequence from the 59th amino acid Gly to the 287th amino acid Ala of SEQ ID NO: 20. Yet another AGPR derivative according to the invention approximately comprises the cytoplasmic domain and extracellular domain of AGPR L-H2. A specific example thereof comprises the cytoplasmic domain having the amino acid sequence from the 1st amino acid Met to the 41st amino acid Ser, and the extracellular domain having the amino acid sequence from the 59th amino acid Gly to the 287th amino acid Ala, of SEQ ID NO: 20.

However, the AGPR derivatives of the invention are not limited to those with the amino acid sequences mentioned above. So long as the antigen determinant site of the AGPR is intact, the AGPR derivatives of the invention also include AGPR derivatives modified by a substitution, deletion and/or addition of one or more amino acids of the above-mentioned amino acid sequences, for example less than 20%, or less than 10%, of the amino acids in each domain, which may be fewer than 6 or fewer than 3 amino acids of the cytoplasmic domain, and fewer than 40, fewer than 20 or fewer than 10 amino acids of the extracellular domain.

The derivatives of the present invention are soluble because they contain no membrane spanning domains, and thus may be easily recovered and purified, while they are also very useful as reagents for assaying anti-AGPR antibodies because they retain the antigen determinant site of AGPR.

The AGPR derivatives of the invention may be in the form of polypeptides without sugar chains or in the form of polypeptides or glycoproteins with sugar chains. The former may be obtained by gene recombination production using bacteria or other prokaryotes, or by chemical synthesis, while the latter may be produced by gene recombination production using animal cells or other eukaryotic cells.

The AGPR derivatives of the invention may be produced according to conventional methods for producing polypeptides, such as chemical synthesis or gene recombination. However, production by gene recombination is preferred considering the size or the molecular weight. In particular, the AGPR derivatives comprising parts of the amino acid sequence of SEQ ID NO: 19 or 20 are preferably produced using human-derived cDNA. AGPR derivatives having amino acid sequences modified by a substitution, deletion and/or addition of one or more amino acids of the amino acid sequence of SEQ ID NO: 19 or 20 may be produced by commonly used site-specific mutagenesis.

cDNA fragments encoding AGPR may be isolated from cDNA synthesized using human liver polyA RNA as the template. The method of isolation may involve using a nucleotide sequence inferred from a published amino acid sequence (references: Spiess et al., J. Biol. Chem. 260; 1979-1982, 1985, Spiess et al., Proc. Natl. Acad. Sci. 82; 6465-6469, 1985) or using part of a published nucleotide sequence, as a probe for screening of a cDNA library synthesized with human liver polyA RNA as the template according to a common method (Current protocols in Molecular Biology: edited by Ausubel et al., Wiley Science; Molecular Cloning: Sambrook et al., Cold Spring Harbor).

According to the present invention, the desired cDNA for H1 was obtained using a primer having part of the nucleotide sequence reported by Spiess et al. (Spiess et al., J. Biol. Chem., 260: 1979-1982, 1985) for synthesis of cDNA with human liver polyA RNA as the template, and amplification by the polymerase chain reaction (PCR). This cDNA was used as the basis to prepare expression vectors for preparation of AGPR polypeptides by a genetic engineering method.

The genetic engineering method used here encompasses any process by which a DNA fragment encoding a peptide with the amino acid sequence represented by any of Sequence Nos. 1 to 4 or a partial sequence thereof is obtained by chemical synthesis, or a combination of techniques. It also encompasses any procedure for constructing a replicable expression vector including a DNA fragment coding for the above-mentioned peptide, a procedure for introducing the expression vector in host cells to obtain a transformant which expresses the peptide, an expression step whereby the transformant is cultured to obtain the expressed product, and a step of recovering the expressed peptide.

Expression vectors to be used for expression in the genetic engineering method may be, in addition to those cited in the examples, vectors employing *E. coli* tryptophan operon as well as tac promoter, tac promoter, lac promoter, PhoA promoter, λ-phage promoter, etc., in the case of an *E. coli* host. Yeast may also be used as the host instead of *E. coli*, for expression using an expression vector including a promoter commonly used in yeast, for example a glycolytic gene, such as for glyceroaldehyde-3-phosphate dehydrogenase, alcohol dehydrogenase I and II, pyruvate kinase, phosphoglycerine kinase, triose phosphate isomerase, etc.

The expression may also be accomplished using an expression system with animal cells, for example mammalian cells such as CHO cells, COS-1 cells or HeLa cells, as the host, with a commonly used integrating expression vector or vaccinia virus as the vector, or an expression system employing adenovirus or another commonly used virus as the vector.

When a genetic engineering method is employed, it is also quite common to express the AGPR peptide as a fused protein with another peptide.

According to the invention, the H1 and L-H2 polypeptides were expressed as fused proteins with a portion of an *E. coli* TrpE gene product. For more efficient expression and purification, they were expressed as H1EM and L-H2EM comprising each of the cellular domain sequences for AGPR H1 and L-H2, and as H1TIM(-) and L-H2TM(-) polypeptides prepared lacking the respective membrane spanning domains and fusing the intracellular domain and extracellular domain.

For expression of the proteins having the same modifications as AGPR expressed by human liver, the recombinant CHO cell line 9801 was prepared which simultaneously expresses H1 and L-H2, for an expression system using mammalian cells as the host. This cell line expresses H1 and L-H2 in a modified form with sugar chains, and the expressed peptides were confirmed to retain receptor activity based on binding to N-galactosamine.

It will become apparent by the examples that detection of anti-AGPR antibodies is possible using recombinant AGPR derivative peptides or cells expressing the recombinant AGPR derivatives, as prepared according to the present invention. Since the antibodies are also found at a high antibody titer in autoimmune hepatitis patients, it will be obvious that detection of the anti-AGPR antibodies is effective as a method of diagnosing autoimmune hepatitis.

The anti-AGPR antibody assay method using AGPR prepared from human liver according to the teaching of Treichel et al. was also investigated on patient serum. The results matched the discrimination results obtained according to the invention, thus demonstrating that AIH patient serum can be discriminated with a system using a peptide prepared according to the invention.

It was also demonstrated by the present invention that anti-AGPR antibodies in patient serum react with both H1 and L-H2 derivative polypeptides and that it largely depends on the epitope present in the extracellular domain. These results indicate that detection of anti-AGPR antibodies in patient serum can be achieved using mostly the extracellular domain as the major component.

Consequently, the present invention provides a method of detecting anti-AGPR antibodies which is characterized by using at least one type of the aforementioned AGPR derivatives. The method involves contacting a test sample such as human or other serum or plasma to be measured for the presence or amount of the anti-AGPR antibodies, with at least one type of AGPR derivative according to the invention, and detecting whether an antigen/antibody reaction occurs between anti-AGPR antibodies in the test sample and the AGPR derivative antigen, or measuring the extent of the antigen/antibody reaction. The system of assay by antigen/antibody reaction is not particularly limited, and any conventional immunoassay method may be used.

The Enzyme-Linked Immunosorbent Assay (ELISA), Western blotting, the indirect fluorescent antibody method, radioimmunoassay (RIA), or the like may be used. The labelling used for the assay method may be an enzyme such as peroxidase, alkali phosphatase, digoxigenase or β-galactosidase, a radioactive isotope such as ¹²⁵I, ³⁵S, ³H or ¹⁴C, or a fluorescent substance such as FITC, rhodamine, Texas red, etc.

The present invention further provides a reagent to be used in the above-mentioned method for assaying anti-AGPR antibodies, which comprises the aforementioned AGPR derivative and either or both AGPR H1 and AGPR L-H2. The AGPR derivative and AGPR in the reagent may be purified or partially purified, or may be the host cells themselves expressing and bearing the polypeptides or proteins. Such a reagent may be used in the form of the AGPR derivative or AGPR dissolved in a buffer solution such as a phosphate buffer, carbonate buffer, Tris-HCl buffer or HEPES buffer solution, one of these solutions which has been dried by lyophilization, the polypeptides or proteins immobilized on a solid carrier such as a filter, glass slide or beads, or the cells themselves expressing and bearing the polypeptide or protein as mentioned above, which have been immobilized on the surface of a filter, glass slide or the like.

The present invention also relates to a kit for assaying anti-AGPR antibodies which comprises at least a reagent such as mentioned above which contains AGPR or AGPR, and standard anti-AGPR antibody. The anti-AGPR antibody may be either polyclonal or monoclonal antibody. The kit sometimes contains a labelled non-human-derived antibody against human immunoglobulin, as in the case of an ELISA kit.

The assay method, assay reagent and assay kit described above may use as the antigen at least one type of AGPR derivative of the invention and AGPR produced by recombination, but it is sometimes preferable for the assay method to employ 2 or more, particularly AGPR H1 or its derivative and AGPR L-H2 or its derivative, in combination. In such cases, cells which express both AGPR H1 or its derivative and AGPR L-H2 or its derivative are preferably used.

Using AGPR or its derivative which can be produced according to the present invention also allows quantitative and qualitative analysis of asialoglycoprotein in blood. Purification of asialoglycoproteins and asialated sugars is also possible.

The peptides of the invention may also be used to develop inhibiting agents against incorporation of asialoglycoprotein through AGPR receptors. They may also be used to develop inhibiting agents against invasion of viruses and complexes of viruses and other proteins into liver cells through AGPR receptors.

The present invention is explained in more detail below by way of the following examples.

### Example 1. Cloning of AGPR H1 cDNA

The gene necessary to construct a Type I AGPR (H1) recombinant was cloned by the PCR from single-stranded cDNA from human liver.

The single stranded cDNA from human liver was prepared from 0.5 µg of human liver poly(A)-RNA (CLONTECH), using a First Strand Synthesis Kit (STRATAGENE). The following protocol was followed. The 0.5 µg of human liver poly(A)-RNA was dissolved in 32 µl of DEPC water, and 0.3 µg/3 µl oligo dT primer was added. After heating the mixture at 65°C for 5 minutes, it was allowed to stand until reaching room temperature.

To this was added 5 µl of a 10x buffer solution, 5 µl of 0.1 M DTT, 1 µl of RNase BlockI, 2 µl of 25 mM dNTPs and 1 µl of MMLVRT (20 U/µl), and after incubation at 37°C for one hour, heating was conducted at 65°C for 15 minutes to inactivate the enzyme and suspend the DNA synthesis reaction. For use in the PCR reaction, the oligo dT primer was removed by a Nick column.

The primers used for the PCR were synthesized from the nucleotide sequence of AGPR H1 determined by M. Spiess et al. (Spiess, M. et al., J. Biol. Chem., 260:1979-1982, 1985), and had the sequences shown below.
Primer F1: 5'-CACTGAAGAACCTGGGAATCAGAC-3' (corresponding to nucleotides 107-130 of SEQ ID NO: 19) (SEQ ID NO: 1)
Primer F1.5: 5'-AGCCCTATCATGACCAAGGAG-3' (corresponding to nucleotides 164-184 of SEQ ID NO: 19) (SEQ ID NO: 2)
Primer B2.5: 5'-CAGGTCGAGGCATTGAAGA-3' (corresponding to nucleotides 1073-1055 of SEQ ID NO: 19) (SEQ ID NO: 3)
Primer B9: 5'-TTTCAAGCTCCTCACCTTCGG-3' (corresponding to nucleotides 1198-1178 of SEQ ID NO: 19) (SEQ ID NO: 4)

Due to the small amount of the gene of interest in the single stranded cDNA from human liver, the PCR reaction was conducted twice for cloning of the AGPR H1 gene. The first PCR reaction employed the following composition.

| | |
|---|---|
| 10x buffer solution (Ampli Taq PCR kit) | 10 µl |
| 2.5 mM dATP | 2 µl |
| 2.5 mM dCTP | 2 µl |
| 2.5 mM dGTP | 2 µl |
| 2.5 mM dTTP | 2 µl |
| 10 pmol/µl primer F1 | 10 µl |
| 10 pmol/µl primer B9 | 10 µl |
| Human liver single stranded cDNA | 5 µl |
| Sterilized water | 56 µl |
| 10 U/µl Taq polymerase | 1 µl |

The reaction was conducted in 35 cycles of 30 seconds at 94°C, 1 minute at 50°C and 2 minutes at 72°C, followed by incubation at 72°C for 7 minutes and preservation at 4°C until the next procedure.

A 50 µl portion of the first PCR reaction solution was subjected to 1.2% agarose gel electrophoresis with a 1xTAE buffer solution, and an approximately 1.1 kb band was cut out. The DNA was recovered from this using Gene Clean (BIO 101), and 1 µl of the recovered 10 µl solution was used for the second PCR reaction. The second PCR reaction employed the following composition.

| | |
|---|---|
| 10x buffer solution | 10 µl |
| 2.5 mM dATP | 2 µl |
| 2.5 mM dCTP | 2 µl |
| 2.5 mM dGTP | 2 µl |
| 2.5 mM dTTP | 2 µl |
| 10 pmol/µl primer F1 | 10 µl |
| 10 pmol/µl primer B9 | 10 µl |
| 1.1 kb DNA fragments obtained by 1st PCR | 1 µl |
| Sterilized water | 60 µl |
| 10 U/µl Taq polymerase | 1 µl |

The reaction was conducted in the same manner as the first. Four µl of the reaction solution was separated by electrophoresis on a 2% agarose gel, the darkest band corresponding to approximately 1.1. kb was cut out and the DNA recovered with Gene Clean. A 3.5 µl portion of the recovered 10 µl solution and 1 µl of pGEM-T vector (Promega) were subjected to ligation using 60 µl of solution A and 7.5 µl of solution B from a ligation kit (Takara Shuzo), and the DNA fragments obtained by PCR were cloned at the multicloning sites of pGEM-T. Four µl of the ligation reaction solution was mixed with 100 µl *E. coli* DH5α competent cells, and heating at 42°C for 30 seconds was followed by rapid cooling to transform the DH5α. The transformed DH5α was looped onto a 1.5% agar LB plate containing 100 µg/ml ampicillin and cultured overnight at 37°C.

The ampicillin-resistant transformants which had formed colonies on the LB plate containing ampicillin were collected with a pick and were planted into 3 ml of 2 × YT medium and cultured overnight at 37°C, after which the transformants were collected and the plasmid DNA recovered by the alkalilysis method. The obtained plasmid DNA was cut with restriction endonuclease, separated by agarose gel electrophoresis, and the lengths thereof were determined to select a clone in which the desired AGPR H1 gene had been cloned in pGEM-T.

The transformant carrying this clone was further planted in 100 ml of 2 × YT medium and cultured overnight at 37°C, and the transformant cells were collected to prepare a large amount of the plasmid DNA by the alkalilysis method. The nucleotide sequence of the inserted portion was confirmed with a fluorescent sequencer (ABI Co.). The clone obtained in this manner has the sequence corresponding to bases 107 to 1198 of the reported AGPR H1 cDNA sequence at the multicloning site of cloning vector pGEM-T, and it was named pGEM-T-H1.

Also, 1/100 µl of the first PCR reaction solution was used for a separate second PCR reaction. This reaction solution had the following composition.

| | |
|---|---|
| 10x buffer solution | 10 µl |
| 2.5 mM dATP | 2 µl |
| 2.5 mM dCTP | 2 µl |
| 2.5 mM dGTP | 2 µl |
| 2.5 mM dTTP | 2 µl |
| 10 pmol/µl primer F1.5 | 10 µl |
| 10 pmol/µl primer B2.5 | 10 µl |
| 1/100 dilution of first PCR solution | 1 µl |
| Sterilized water | 60 µl |
| 10 U/µl Taq polymerase | 1 µl |

The reaction was conducted in 20 cycles of 30 seconds at 94°C, 1 minute at 60°C and 2 minutes at 72°C, followed by reaction for 7 minutes at 72°C. Forty µl of the reaction solution was separated by electrophoresis on a 2% agarose gel, and a band corresponding to a size of approximately 900 bases was cut out. The DNA was recovered from the gel fragments with Gene Clean Kit[BIO 101], and 3.5 µl of the recovered 10 µl DNA solution and 5 µl of pGEM-T vector were subjected to ligation using 60 µl of solution A and 7.5 µl of solution B from a Takara Shuzo ligation kit. Selection was thus made of desired gene cloned in pGEM-T and having bases 164 to 1073 of the reported AGPR H1 cDNA sequence.

Of the obtained pGEM-T-H1 (164-1073), the PmaCI restriction enzyme recognition site of the insert and the SalI recognition site at the 3'-end pGEM-T multicloning site of the insert were recombined with PmaCI/SalI of pGEM-T-H1 to prepare clone pGEM-T-H1-2 having the sequence from bases 164 to 1198 of the reported AGPR H1 cDNA sequence.

### Example 2. Cloning of AGPR L-H2 cDNA

The gene necessary to construct a Type II AGPR (L-H2) recombinant was cloned by the PCR from single stranded cDNA from human liver, in the same manner as H1 in Example 1.

The primer used for the PCR was synthesized from the nucleotide sequence of AGPR L-H2 determined by M. Spiess et al. (Spiess et al., Proc. Natl. Acad. Sci. 82:6465-6469, 1985, Paietta, E., et al. Hepatology 15:395-402, 1992). The following sequences were used.
Primer 1: 5'-ATCATGGCCAAGGACTTTCAA-3' (corresponding to nucleotides 188-208 of SEQ ID NO: 20) (SEQ ID NO: 5)
Primer 2: 5'-ATGGGTTAGCCAGAGGTGTGCT-3' (corresponding to nucleotides 1080-1059 of SEQ ID NO: 20) (SEQ ID NO: 6)
Primer 3: 5'-ATGGTCTGCTTCAGTCTGCTTGCC-3' (SEQ ID NO: 7) (corresponding to nucleotides 299-322 of SEQ ID NO: 20)
Primer 4: 5'-CTGTCTCAGTGTTTCTTCCTTTCC-3' (SEQ ID NO: 8) (corresponding to nucleotides 1148-1125 of SEQ ID NO: 20)
Primer 7: 5'-CCCCAGTTCTCCTGGCTTTAAC-3' (SEQ ID NO: 9) (corresponding to nucleotides 34-55 of SEQ ID NO: 20)
Primer 8: 5'-GGCATCGTGATCTGCTTTCAGG-3'(SEQ ID NO: 10) (corresponding to nucleotides 553-532 of SEQ ID NO: 20)

Due to the small amount of the gene of interest in the single stranded cDNA from human liver, the PCR reaction was conducted twice for cloning of the AGPR L-H2 gene. The PCR reaction conditions and method of cloning were the same as in Example 1. The cloned and amplified sequence obtained using primer 1 and primer 2 in the PCR reaction corresponds to bases 188-1080 of the L-H2 cDNA. The cloned and amplified sequence obtained using primer 3 and primer 4 in the PCR reaction corresponds to bases 299-1148 of the L-H2 cDNA. The cloned and amplified sequence obtained using primer 7 and primer 8 in the PCR reaction corresponds to bases 34-553 of the L-H2 cDNA. All of these were cloned in the same direction in pGEM-T. The clones cloned in pGEM-T were dubbed pGEM-T-LH2-12, pGEM-T-LH2-34 and pGEM-T-LH2-5', respectively.

The XhoI/SphI region of pGEM-T-LH2-34 (299-1148) was recombined with the XhoI/SphI (3' end multicloning site) of pGEM-T-LH2-12 (188-1080) to construct pGEM-T-L-H2 (188-1148). Also, the SphI/EcoRV region of pGEM-T-LH2-5' (34-553) was recombined with SpeI (5' end multicloning site) of pGEM-T-L-H2 to construct pGEM-T-L-H2F. This clone includes bases 34 through 1148 of the reported L-H2 cDNA.

### Example 3. Expression of AGPR H1 protein extracellular domain by AGPR H1 gene-recombinant E. coli

For expression of the extracellular domain of the AGPR H1 protein in *E. coli*, the gene for the extracellular domain of AGPR H1 was cloned and inserted into the expression vector pAT-TrpE.

The cloning of the extracellular domain of the AGPR H1 gene was accomplished by the PCR using pGEM-T-H1 as the template, cloning the amplified DNA in pGEM-T. The following PCR primers were used.
Primer AG1EcoF: 5'-GAATTCGGATCCCAAAACTCCCAG-3'(SEQ ID NO: 11) (nucleotides 350-367 of SEQ ID NO: 19)
Primer AG1SalR: 5'-GTCGACTTAAAGGAGAGGTGGCTC-3'(SEQ ID NO: 12) (nucleotides 1048-1031 of SEQ ID NO: 19)

Primers AG1EcoF and AG1SalR include the EcoRI restriction enzyme recognition site and the SalI recognition site, respectively, and the H1 extracellular domain gene amplified by the two primers has the EcoRI recognition site at the 5' end and the SalI recognition site at the 3' end. The PCR reaction was conducted using the following composition.

| | |
|---|---|
| 10x buffer solution | 10 µl |
| 2.5 mM dATP | 2 µl |
| 2.5 mM dCTP | 2 µl |
| 2.5 mM dGTP | 2 µl |
| 2.5 mM dTTP | 2 µl |
| 10 pmol/µl primer AG1EcoF | 10 µl |
| 10 pmol/µl primer AG1SalR | 10 µl |
| pGEM-T-H1 | 0.5 µl |
| Sterilized water | 60.5 µl |
| 10 U/µl Taq polymerase | 1 µl |

The reaction was conducted in 25 cycles of 30 seconds at 94°C, 1 minute at 45°C and 2 minutes at 72°C, followed by reaction for 7 minutes at 72°C. An approximately 700 b band obtained by the reaction was cut out, subjected to ligation reaction with a ligation buffer solution (Takara Shuzo), and cloned in pGEM-T. The resulting plasmid was dubbed pGEM-T-H1EM. The nucleotide sequence was determined using restriction endonuclease and sequencing, and it was then cut at the EcoRI/SalI restriction enzyme recognition site prepared with the PCR primer, and inserted by ligation reaction at the EcoRI/SalI site of pAT-TrpE. The resulting expression vector was named pAT-TrpE-H1EM, and after confirming the sequence with restriction endonuclease, it was introduced into XL-1 Blue competent cells to obtain transformants.

The extracellular domain of AGPR H1 protein was expressed as a fused protein with a polypeptide consisting of the 17 N-terminal amino acids of TrpE. Expression of the protein of interest was confirmed in the following manner. pAT-TrpE-H1EM/XL-1 blue cells were seeded at a 2% concentration in an M9-CA medium containing 100 µg/ml ampicillin, and shake cultured overnight at 37°C. After collecting the *E. coli* cultured overnight, they were subjected to SDS-PAGE according to conventional methods to separate the protein, and then stained with Coomassie Brilliant Blue R250 solution to detect the protein.

As a result, a protein band not detected with the XL-1 blue was detected in with pAT-TrpE-H1Em/XL-1 blue, and the darkness of the band showed that it was expressed in a large amount compared to the other proteins detected in *E. coli*. The molecular weight of the protein according to SDS-PAGE was about 30 K, and this roughly equaled the calculated 28 K molecular weight for the TrpE-H1EM protein. The major band detected by SDS-PAGE was thus judged to be the TrpE-AGPR H1 extracellular domain fused protein, which was the protein of interest. The protein was dubbed H1EM.

In order to obtain H1EM in a large amount, culturing was conducted on a 1L scale. The pAT-TrpE-H1EM/XL-1 blue colonies were seeded into 2 ml 2 × YT/100 µg/ml ampicillin and shake cultured for 8 hours at 37°C. This was reseeded into 30 ml 2 × YT/100 µg/ml ampicillin at a proportion of 1/50 and shake cultured at 37°C overnight. On the following day, pAT-TrpE-H1Em XL-1 blue cells cultured in 1 L M9-CA/100 µg/ml ampicillin at a proportion of 1/50 were seeded and shake cultured at 37°C overnight. After confirming expression of the H1EM protein, the cells were collected by centrifugation and the resulting pellet (*E. coli*) was cryopreserved at -20°C.

The H1EM protein was purified for use in the subsequent experiment. One gram of the cryopreserved *E. coli* was suspended in 5 ml of dissolving buffer solution (50 mM Tris-HCl, pH 8.0/30 mM NaCl/5 mM EDTA), and 1 mg of lysozyme was added prior to shaking at 27°C for one hour. After sonication twice at 150 W for 90 seconds, the cells were disrupted. The disruption of the cells was confirmed by microscope. These were separated by centrifugation at 15K, 4°C for 30 minutes to obtain a pellet which was then suspended by addition of buffer A (50 mM Tris-HCl, pH 8.0) and homogenized at 1500 rpm, 5 strokes.

The pellet obtained by subsequent centrifugation at 15K, 4°C for 15 minutes was washed with buffer A and again subjected to centrifugation. The resulting pellet was suspended by addition of buffer A containing 2 M urea, and was then centrifuged at 15K, 4°C for 15 minutes to obtain a supernatant and a pellet. The pellet was also subjected to the same procedure using 4M and 6M urea. After urea extraction, the extract obtained by each procedure was subjected to SDS-PAGE electrophoresis and the protein was detected by CBB staining, which demonstrated the presence of the TrpE-H1EM fused protein. The protein of interest was present in the 4-6 M urea extraction solutions together with contaminants.

The 4-6 M urea extraction solutions were passed through Q Sepharose (Pharmacia) (16/10) and eluted with 0 to 0.3 M NaCl for 15 minutes at a flow rate of 0.4 ml/min. The eluted fractions exhibiting an absorption peak at O.D. 280 nm were passed through Sephacryl S-300 (Pharmacia) (26/95) and eluted with buffer B (20 mM Tris-HCl, pH 8.0/6 M urea/0.1 M NaCl/5 mM DTT) at a flow rate of 2 ml/min. The eluted fractions were again passed through Sephacryl S-300 (Pharmacia) (26/95) to obtain eluted fractions exhibiting absorption at O.D. 280 nm in fractions 120-160.

Separation and confirmation of the protein by SDS-PAGE revealed a single band of protein, though with some contaminants. The DTT was removed from the buffer solution of the eluted fractions containing the desired protein using Sephadex G25 fine according to the manual, the solution was concentrated with Centricon-10 (Amicon) according to the manual, and the buffer solution was replaced with PBS(-) in a PD-10 column (Pharmacia) also according to the manual. The purified H1EM peptide was thus obtained.

### Example 4. Expression of fused protein of cytoplasmic and extracellular domains of AGPR H1 protein by AGPR H1 gene-recombinant E. coli

In order to express the fused protein of the AGPR H1 protein cytoplasmic and extracellular domains, the cytoplasmic and extracellular domains of the AGPR H1 gene were cloned and linked together prior to insertion in expression vector pTac-TrpE.

The gene cloned in Example 3 was used as the extracellular domain of the AGPR H1 gene. The cloning of the cytoplasmic domain of the AGPR H1 gene was accomplished by PCR reaction with pGEM-T-H1 as the template, and cloning the amplified DNA in pGEM-T. The following PCR primers were used.
Primer H1XhoF: 5'-CTCGAGATGACCAAGGAGTATCAAG-3' (SEQ ID NO: 13) (nucleotides 173-191 of SEQ ID NO: 19)
Primer H1EcoR: 5'-GAATTCGAGGAGGCGAGGTCCGGA-3' (SEQ ID NO: 14) (nucleotides 298-281 of SEQ ID NO: 19)

Primers H1XhoF and H1EcoR include the XhoI restriction enzyme recognition site and the EcoRI recognition site, respectively, and the H1 extracellular domain gene amplified by the two primers has the XhoI recognition site at the 5' end and the EcoRI recognition site at the 3' end. The PCR reaction was conducted using the following composition.

| | |
|---|---|
| 10x buffer solution | 10 µl |
| 2.5 mM dATP | 2 µl |
| 2.5 mM dCTP | 2 µl |
| 2.5 mM dGTP | 2 µl |
| 2.5 mM dTTP | 2 µl |
| 10 pmol/µl primer H1XhoF | 10 µl |
| 10 pmol/µl primer H1EcoR | 10 µl |
| pGEM-T-H1 | 0.5 µl |
| Sterilized water | 60.5 µl |
| 10 U/µl Taq polymerase | 1 µl |

The reaction was conducted in 10 cycles of 30 seconds at 94°C, 1 minute at 45°C and 1 minute at 72°C, followed by reaction for 7 minutes at 72°C. An approximately 127 b band obtained by the reaction was cut out, subjected to ligation reaction with a ligation buffer solution (Takara Shuzo), and cloned in pGEM-T. The resulting plasmid was dubbed pGEM-T-H1IM. The base sequence was determined by confirming the restriction enzyme cleavage sites and by sequencing, and it was then cut at the XhoI/EcoRI restriction enzyme recognition sites prepared with the PCR primer.

The XhoI/EcoRI-cut fragments of pGEM-T-H1IM and EcoRI/SpeI-cut fragments of the pGEM-T-H1EM obtained in Example 3 were inserted by ligation reaction at the XhoI/SpeI site of the *E. coli* expression vector pTac-TrpE including the Tac promoter. The resulting expression vector was named pTac-H1TM(-), and after confirming the sequence with restriction endonuclease, it was introduced into XL-1 Blue competent cells to obtain transformants.

The fused protein of the H1 cytoplasmic and extracellular domains was expressed as a fused protein with a polypeptide consisting of the 17 N-terminal amino acids of TrpE. Expression of the protein of interest was confirmed in the following manner. pTac-H1TM(-)/XL-1 blue cells were seeded at a 2% concentration in LB medium containing 100 µg/ml ampicillin, and shake cultured at 37°C until a value of 40 was reached with a turbidimeter. After addition to the culture solution of IPTG at a final concentration of 0.4 mM, culturing was continued for 6 hours and the *E. coli* cells were collected. After collecting the *E. coli*, they were subjected to SDS-PAGE according to conventional methods to separate the protein and then stained with Coomassie Brilliant Blue R250 (CBB) solution to detect the protein.

As a result, a protein band not detected with the XL-1 blue was detected in with pTac-H1TM(-)/XL-1 blue, and the darkness of the band showed that it was expressed in a large amount compared to the other proteins detected in *E. coli*. The molecular weight of the protein according to SDS-PAGE was about 35 K, and this roughly equaled the calculated 34 K molecular weight for the TrpE-H1TM(-) protein. The major band detected by SDS-PAGE was thus judged to be the TrpE-H1 cytoplasmic and extracellular domain fused protein, which was the protein of interest. The protein was dubbed H1TM(-).

In order to obtain H1TM(-) in a large amount, culturing was conducted on a 2L scale. The pTac-H1TM(-)/XL-1 blue colonies were seeded into 30 ml LB/100 µg/ml ampicillin and shake cultured overnight at 37°C. On the following day, pTac-H1TM(-)/XL-1 blue cells cultured in 2 L LB/100 µg/ml ampicillin at a proportion of 1/100 were seeded and shake cultured at 37°C for 2 hours, after which IPTG at a final concentration of 0.4 mM was added and the culturing was continued overnight. After confirming expression of the H1TM(-) protein, the cells were collected by centrifugation and the resulting pellet (*E. coli*) was cryopreserved at -20°C.

The H1TM(-) protein was purified for use in the subsequent experiment. Urea extraction was performed in the same manner as Example 3. Five ml of 1% NP-40 was added to one gram of the *E. coli* in buffer A. The protein of interest was present in the 6 M urea extraction solution together with contaminants. DTT at a final concentration of 50 mM was added to the 6 M urea extraction solution which was then allowed to stand at 4°C for over an hour. This was passed through previously equilibrized Q-Sepharose (Pharmacia) (16/10) and eluted with 0-0.3 M NaCl in buffer C (50 mM Bis-Tris/6 M urea/5 mM DTT/pH 7.0 with HCl) at a flow rate of 2 ml/min.

The 0.15-0.2 M NaCl elution fraction exhibited an absorption peak at O.D. 280 nm. Upon separation and identification of the protein by SDS-PAGE, almost a single band of protein was detected. The eluted fraction containing the desired protein was concentrated with an Amicon YM10 according to the manual, and the DTT was brought to a low concentration with buffer C lacking DTT. The purified H1TM(-) peptide was thus obtained.

### Example 5. Expression of extracellular domain of AGPR L-H2 protein by AGPR L-H2 gene-recombinant E. coli

For expression of the extracellular domain of L-H2 protein as well in *E. coli* in the same manner, the extracellular domain of the AGPR L-H2 gene was cloned and inserted into expression vector pAT-TrpE.

The cloning of the extracellular domain of the AGPR L-H2 gene was accomplished by PCR reaction using pGEM-T-L-H2 as the template, in the same manner as the cloning of the H1 extracellular domain. The following PCR primers were used.
Primer AGEcoF: 5'-GAATTCGGGTCCCAAAGTGCACAG-3' (SEQ ID NO: 15) (nucleotides 365-382 of SEQ ID NO: 20)
Primer AGEcoR: 5'-GGATCCTTATCAGGCCACCTCGCCGGT-3' (SEQ ID NO: 16) (nucleotides 1054-1037 of SEQ ID NO: 20)

Primers AGEcoF and AGEcoR include the EcoRI restriction enzyme recognition site and the BamHI recognition site, respectively, and the L-H2 extracellular domain gene amplified by the two primers has the EcoRI recognition site at the 5' end and the BamHI recognition site at the 3' end. The PCR reaction was conducted in the same manner as for H1.

The procedure after the PCR reaction was also the same as for H1, and the plasmid obtained by inserting the PCR product in vector pGEM-T was named pGEM-T-L-H2EM. The L-H2 extracellular domain gene was inserted at EcoRI/BamHI of pAT-TrpE to prepare expression vector pAT-TrpE-L-H2EM. This was introduced into XL-1 Blue competent cells to obtain transformants.

Expression of the fused protein of the 17 N-terminal amino acid polypeptide of TrpE and the L-H2 extracellular domain was confirmed in the same manner as expression of the TrpE-H1EM fused protein.

As a result, a protein band not detected with the XL-1 blue was detected with pAT-TrpE-L-H2EM/XL-1 blue, and the darkness of the band showed that it was expressed in a large amount compared to the other proteins detected in *E. coli*. The molecular weight of the protein according to SDS-PAGE was about 30 K, and this roughly equaled the calculated 28 K molecular weight for the TrpE-L-H2EM protein. The major band detected by SDS-PAGE was thus judged to be the TrpE-L-H2 extracellular domain fused protein, which was the protein of interest. The protein was named L-H2EM.

In order to obtain the L-H2EM protein in a large amount, culturing was conducted on a 1L scale. The pAT-TrpE-L-H2EM/XL-1 blue colonies were seeded into 2 ml 2 × YT/100 µg/ml ampicillin and shake cultured for 8 hours at 37°C. This was reseeded into 30 ml 2 × YT/100 µg/ml ampicillin at a proportion of 1/50 and shake cultured at 37°C overnight. On the following day, pAT-TrpE-L-H2EM XL-1 blue cells cultured in 1 L M9-CA/100 µg/ml ampicillin at a proportion of 1/50 were seeded and shake cultured at 37°C overnight. After confirming expression of the L-H2EM protein, the cells were collected by centrifugation and the resulting pellet (*E. coli*) was cryopreserved at -20°C.

The L-H2EM protein was purified for use in the subsequent experiment. Urea extraction was performed in the same manner as Example 3. The protein of interest was present in the 4-6 M urea extraction solution, together with contaminants. The 4-6 M urea extraction solution was passed through Q Sepharose (Pharmacia) (16/10) and eluted with 0-0.3 M NaCl for 15 minutes at a flow rate of 0.4 ml/min. The eluted fractions exhibiting an absorption peak at O.D. 280 nm were passed through Sephacryl S-300 (Pharmacia) (26/95) and eluted with buffer B at a flow rate of 2 ml/min. The eluted fractions were passed through Superdex 75 pg (26/60) and eluted with buffer D (20 mM Tris-HCl, pH 8.0/6 M urea/0.1 M NaCl/5 mM DTT) at a flow rate of 2 ml/min.

The protein of interest eluted in fractions 55-65. Separation and identification of the protein by SDS-PAGE of the eluted fraction revealed almost a single band of protein. The DTT was removed from the buffer solution of the eluted fractions containing the desired protein using Sephadex G25 fine, the solution was concentrated with Centricon-10 (Amicon), and the buffer solution was replaced with PBS(-) in a PD-10 column (Pharmacia) according to the manual. The purified L-H2EM peptide was thus obtained.

### Example 6. Expression of fused protein of cytoplasmic and extracellular domains of AGPR L-H2 protein by AGPR L-H2 gene-recombinant E. coli

For expression of the linked cytoplasmic and extracellular domains of L-H2 protein as well in *E. coli* in the same manner, the cytoplasmic and extracellular domains of the AGPR L-H2 gene were cloned and inserted into expression vector pAT-TrpE.

The cloning of the cytoplasmic domain of the AGPR L-H2 gene was accomplished by PCR reaction using pGEM-T-L-H2 as the template, in the same manner as the cloning of the H1 cytoplasmic domain. The following PCR primers were used.
Primer L-H2XhoF: 5'-CTCGAGATGGCCAAGGACTTTCAA-3' (SEQ ID NO: 17) (nucleotides 191-208 of SEQ ID NO: 20)
Primer L-H2EcoR: 5'-GAATTCACTGAAGCAGACCATGGAG-3' (SEQ ID NO: 18) (nucleotides 313-295 of SEQ ID NO: 20)

Primers L-H2XhoF and L-H2EcoR include the XhoI restriction enzyme recognition site and the EcoRI recognition site, respectively, and the L-H2 extracellular domain gene amplified by the two primers has the XhoI recognition site at the 5' end and the EcoRI recognition site at the 3' end. The PCR reaction was conducted in the same manner as for H1.

The procedure after the PCR reaction was also the same as for H1, and the plasmid obtained by inserting the PCR-obtained fragment in vector pGEM-T was dubbed pGEM-T-L-H2TM(-). The fragment obtained by cutting pGEM-T-L-H2TM(-) with XhoI/EcoRI restriction enzyme and the fragment obtained by cutting pGEM-T-L-H2EM with EcoRI/BamHI restriction enzyme were inserted at the XhoI/BamHI site of pTac-TrpE to prepare the *E. coli* expression vector pTac-L-H2TM(-). This was introduced into XL-1 Blue competent cells to obtain transformants.

Expression of the fused protein of the 17 N-terminal amino acid polypeptide of TrpE and the L-H2 cytoplasmic and extracellular domain fused protein was confirmed in the same manner as expression of the TrpE-LH1TM(-) fused protein.

As a result, a protein band not detected with the XL-1 blue was detected with pTac-L-H2TM(-)/XL-1 blue, and the darkness of the band showed that it was expressed in a large amount compared to the other proteins detected in *E. coli*. The molecular weight of the protein according to SDS-PAGE was about 35 K, and this roughly equaled the calculated 33 K molecular weight for the TrpE-L-H2TM(-) protein. The major band detected by SDS-PAGE was thus judged to be the TrpE-L-H2 cytoplasmic and extracellular domain fused protein, which was the protein of interest. The protein was named L-H2TM(-).

In order to obtain the L-H2TM(-) protein in a large amount, culturing was conducted on a 2L scale. The pTac-L-H2TM(-)/XL-1 blue colonies were seeded into 30 ml of 2 × YT/100 µg/ml ampicillin and shake cultured overnight at 37°C. On the following day, pTac-L-H2TM(-)/XL-1 blue cells cultured in 2 L LB/100 µg/ml ampicillin at a proportion of 1/100 were seeded and shake cultured at 37°C for 2 hours, after which IPTG at a final concentration of 0.4 mM was added and the culturing was continued for 6 hours. After confirming expression of the L-H2TM(-) protein, the cells were collected by centrifugation and the resulting pellet (*E. coli*) was cryopreserved at -20°C.

The L-H2TM(-) protein was purified for use in the subsequent experiment. Urea extraction was performed in the same manner as Example 3. Five ml of 1% NP-40 was added to one gram of the *E. coli* in buffer A. The protein of interest was present in the 4-6 M urea extraction solution together with contaminants. DTT at a final concentration of 50 mM was added to the 6 M urea extraction solution which was then allowed to stand at 4°C for over an hour. This was passed through equilibrized MonoQ (Pharmacia) (5/5) and eluted with 0-0.3 M NaCl in buffer E (50 mM Tris-HCl, pH 8.0/6 M urea/5 mM DTT) at a flow rate of 1 ml/min.

The approximately 0.1 M NaCl elution fraction exhibited an absorption peak at O.D. 280 nm. Upon separation and identification of the protein by SDS-PAGE, almost a single band of protein was detected. The eluted fraction containing the protein of interest was dialyzed in buffer F (50 mM Tris, pH 8.0/6 M urea/0.15 M NaCl) and the DTT was removed. The purified L-H2TM(-) peptide was thus obtained.

### Example 7. Preparation of anti-AGPR H1 antibody and anti-AGPR L-H2 antibody

The purified recombinant AGPR proteins H1EM and L-H2Em obtained above in Examples 3 and 5 were used to prepare anti-AGPR H1 antibody and anti-AGPR L-H2 antibody. Rabbits (New Zealand white) and mice were used for the immunization, and Freund's complete adjuvant (FCA) was used as the adjuvant for immunization according to conventional methods. The antibody titer was measured by ELISA, and the reaction specificity was confirmed by Western blotting.

ELISA was conducted according to the following method. The H1EM purified protein or L-H2EM purified protein was coated on a plate (Nunc) to 1 µg/ml at room temperature for 2 hours. Appropriately diluted serum was then incubated at room temperature for one hour. Peroxidase-bound anti-rabbit IgG was used at 1000-fold dilution as the secondary antibody and incubation was conducted at room temperature for one hour. Coloring was accomplished by incubation at room temperature for 15 minutes in a mixture of 6 µl hydrogen peroxide water and a solution of an OPD1 tablet (Wako Junyaku) in 35 ml of phosphate/citrate buffer solution, and an equivalent of 10% sulfuric acid was added to suspend the reaction. The detection was accomplished by measurement at OD 492 nm with a microplate reader (Tori).

The Western blotting was conducted according to conventional methods. SDS-PAGE was performed according to conventional methods using XL-1 blue cells expressing H1EM or L-H2EM, and wild XL-1 blue cells as the samples. These were then transferred onto a PVDF membrane (millipore) by a conventional method. Each of the antiserums was used at 10,000-fold dilution as the primary antibody, and alkali-phosphatase-bound anti-rabbit IgG was used at 1000-fold dilution as the secondary antibody, for Western blotting according to conventional methods.

The AGPR protein in the antiserums obtained as a result could be detected by ELISA and Western blotting The anti-H1 antiserum exhibited an antibody titer of 10⁵ against H1EM antigen in ELISA. The anti-L-H2 antiserum also exhibited an antibody titer of 10⁵ against L-H2EM antigen. In Western blotting, the anti-H1 antiserum exhibited strong reactivity to AGPR H1 protein while the anti-L-H2 antiserum also exhibited strong reactivity to AGPR L-H2 protein, and despite crossing the reactions, each was found to be specific antiserum.

The reaction between the AGPR proteins expressed by the cultured animal cells obtained in Examples 8 to 12 and the rabbit antiserum obtained in Example 7 was then investigated. As a result of Western blotting of the AGPR proteins expressed by the cultured animal cells at 1000-fold, and by the indirect fluorescent antibody method at up to 50-fold, the antibodies against each subtype were shown to exhibit no cross-reactivity against antigens of different subtypes.

### Example 8. Expression of AGPR H1 protein in cells of the established mammalian cell line COS-1

Cells of the established mammalian cell line COS-1 were transfected with an expression vector in which he AGPR H1 gene had been inserted, to express the AGPR H1 protein in the COS-1 cells. The method of expressing the AGPR H1 protein in the COS-1 cells is described below.

Plasmid PaSR was used as the vector for expression in the COS-1 cells. Five µg of PaSR DNA was cut with 25 U XhoI and 25 U BamHI and treated with 5 U of Klenow fragment according to conventional methods to smooth the ends. It was further treated with 10 U BAP according to conventional methods for dephosphorylation of the ends.

H1 (nucleotides 107-1198) cut from pGEM-T-H1 with SacI/SacII and H1-2 (nucleotides 164-1198) cut out from pGEM-T-H1-2 with SacI/SacII were used as the two AGPR H1 genes for insertion into the expression vector. In both cases, the expression units were cut out by cutting 10 µg of plasmid with 50 U SacI and 50 U SacII, and the ends were smoothed using a branching kit (Takara Shuzo) according to the attached protocol.

The vectors and H1 and H1-2 fragments prepared above were ligated using a ligation kit (Takara Shuzo) according to the attached protocol. The reaction solution was introduced into competent cells by a conventional method for transformation, and a few ampicillin-resistant colonies were obtained. The plasmids were then extracted by a conventional method and cut with restriction endonuclease and identified to obtain the desired expression vectors PaSR-H1 and PaSR-H1-2.

The expression vectors were introduced into COS-1 cells by transfection using electroporation. Growth-phase COS-1 cells cultured overnight were suspended in PBS(-) to a concentration of 2.5 × 10⁶ cells/ml. A 0.6 ml portion of the cell suspension was placed in an electroporation cuvette (Bio-Rad), mixed with 15 µg of expression vector and allowed to stand in ice for 30 minutes. Electroporation was then conducted at 125 µF, 125 V. The cells were allowed to stand in ice for 10 minutes and then seeded into a 100 mmΦ tissue culturing dish containing DMEM 10% FBS. After culturing for 24 hours the medium was replaced, and after 48 hours of electroporation the cells were collected.

The expressed proteins were detected by Western blotting. The recovered cells were suspended in SDS-sample buffer according to a conventional method and an amount of 1 × 10⁵ cells per lane was separated by SDS-PAGE. After transfer of the protein to a PVDF membrane by a conventional method, the anti-H1 serum obtained in Example 6 was used at a 1000-fold dilution for Western blotting by conventional methods. Detection was accomplished using an ECL Western blotting biotin system (Amersham).

As a result, no band was detected at the position just under 46K molecular weight with the COS-1 cells, but with the transfected COS-1 cells a band was observed for the protein being detected. Although the calculated molecular weight of AGPR H1 protein is 38K, the molecular weight with linked sugar chains has been reported to be about 46K with SDS-PAGE. Thus, the band detected by this Western blotting was judged to be the glycosilated AGPR H1 protein.

The methods described above were used to express the AGPR H1 protein in the COS-1 cells to obtain AGPR H1 protein expressed by COS-1 cells.

### Example 9. Expression of AGPR L-H2 protein in cells of the established mammalian cell line COS-1

Cells of the established mammalian cell line COS-1 were transfected with an expression vector in which the AGPR L-H2 gene had been inserted, to express the AGPR L-H2 protein in COS-1 cells. The expression of the AGPR L-H2 protein in the COS-1 cells was accomplished in the same manner as Example 8.

LH-2 (nucleotides 188-1148) cut from pGEM-T-L-H2 with SpeI/SphI and L-H2F (nucleotides 34-1148) cut out from pGEM-T-L-H2F with SpeI/SfiI were used as the two AGPR LH-2 genes for insertion into the expression vector. In both cases, the expression units were cut out by cutting 10 µg of plasmid with 50 U SpeI, 50 U SphI or 50 U SfiI, and the ends were smoothed using a branching kit (Takara Shuzo) according to the attached protocol.

The ligation of the fragments and transformation of *E. coli* and were accomplished in the same manner as Example 8 to obtain a few ampicillin-resistant colonies. The plasmids were then extracted by a conventional method and cut with restriction endonuclease and identified to obtain the desired expression vectors PaSR-L-H2 and PaSR-L-H2F.

Introduction of the expression vectors into COS-1 cells was accomplished by transfection using lipofectin (GIBCO BRL). The COS-1 cells in an amount of 5 × 10⁵ were seeded into a 100 mmΦ tissue culturing dish and cultured overnight. Two µg of DNA prepared according to the lipofectin manual was mixed therewith also according to the manual, and further culturing was conducted overnight. 10% FBS was then added to the medium, and after 48 hours the cells were collected.

The expressed protein was detected by Western blotting in the same manner as the COS-1-expressed H1 protein in Example 8. Anti-L-H2 antiserum diluted 1000-fold was used as the primary antibody.

As a result, for transfection with both PaSR-L-H2 and PaSR-L-H2F, as with PaSR-H1, no band was detected at the position just under 46K molecular weight with the COS-1 cells, but with the transfected COS-1 cells a band was observed for the protein being detected. Although the calculated molecular weight of AGPR L-H2 protein is 38K, the molecular weight with linked sugar chains has been reported to be about 46K with SDS-PAGE. Thus, the band detected by this Western blotting was judged to be the glycosilated AGPR L-H2 protein.

The methods described above were used to express the AGPR L-H2 protein in the COS-1 cells to obtain AGPR L-H2 protein expressed by COS-1 cells.

### Example 10. Expression of AGPR H1 protein in cells of the established cell line CHO-K1

The AGPR H1 gene was introduced into cells of the established animal cell line CHO-K1 to express AGPR H1 protein. The method of expressing the AGPR H1 protein in the CHO-K1 cells is described below.

The expression vector for introduction of the AGPR H1 gene into the CHO-K1 cells was constructed according to the Marcos method (Saito, et al., Experimental Medicine, Vol.7, p.183-). A 30 µg portion of expression vector PaSR-H1 obtained in Example 8 was cut with 200 U of restriction enzyme SfiI, and the resulting DNA fragments were recovered with a DNA CELL (Daiichi Kagaku). A 1.5 µg portion of vector pCHD2L carrying ampicillin-resistant and hygromycin B-resistant genes was also cut with 20 U SfiI, and phenol/chloroform extraction and ethanol precipitation were followed by dissolution in TE (pH 7.5).

Fifty ng of pCHD2L digested with restriction enzyme SfiI and 1 µg of the PaSR-H1 Sfi fragments were dissolved in 6 µl of 100 mM Tris-HCl, pH 7.6/5 mM MgCl₂/300 mM NaCl, mixed with a equivalent of ligation buffer B (Takara Shuzo) and subjected to ligation at 15°C overnight. This produced an approximately 48K Marcos DNA. Using restriction enzyme digestion at KpnI and restriction enzyme digestion at SfiI present on pCHD2L and restriction enzyme digestion at SpeI present in the SfiI fragment of PaSR-H1, it was confirmed that the obtained Marcos DNA had a plurality (about 20) of the Sfi fragments of PaSR-H1 introduced in the same direction per pCHD2L vector.

The Marcos DNA clones with the largest molecular weight were selected, and 30 µg of plasmid prepared with Quagen tip-500 (Quagen) was introduced into 5 × 10⁵ CHO-K1 cells by the calcium phosphate method using a Mammalian Transfection Kit (STRATAGENE). On the 48th hour after transfection, the cells were relooped at 5 × 10⁵ per 100 mmΦ tissue culturing dish, and 600 or 800 µg of hygromycin B was added and selection was made of the CHO-K1 lines which exhibited hygromycin B resistance due to incorporation of the foreign gene into the genome. The selection was conducted for about 2 weeks thereafter. A few colonies of CHO-K1 cells exhibiting hygromycin B resistance were cloned, and expression of AGPR H1 protein was confirmed by Western blotting as explained in Example 8.

The clones confirmed to express AGPR H1 protein were also verified by the indirect fluorescent antibody method. The indirect fluorescent antibody method was conducted as follows. After culturing 1 × 10⁴ cells for 3 days on a chamber slide and washing with PBS(-), they were immobilized with acetone:methanol=1:1, and air-dried. The immobilized cells and anti-H1 serum diluted 100-fold with PBS(-) were incubated for 30 minutes at 37°C, and after washing with PBS(-), they were further incubated with anti-rabbit Igs-fluorescein for 30 minutes at 37°C and again washed with PBS(-). The fluorescence was observed with a fluorescent microscope.

As a result, clones were obtained for which expression of AGPR H1 protein was confirmed by Western blotting and expression of AGPR H1 protein was also confirmed by the indirect fluorescent antibody method in virtually 100% of the cells. The AGPR H1 protein was detected on the cell surfaces and cytoplasm.

### Example 11. Expression of AGPR L-H2 protein in cells of the established cell line CHO-K1

The AGPR L-H2 gene was introduced into cells of the established animal cell line CHO-K1 to express AGPR L-H2 protein. The method of expressing the AGPR L-H2 protein in the CHO-K1 cells is described below.

The expression vector for introduction of the AGPR L-H2 gene into the CHO-K1 cells was constructed by the Marcos method as for AGPR H1 in Example 10. To prepare the L-H2 gene as the expression unit, 30 µg of expression vector PaSR-L-H2 obtained in Example 9 was cut with 200 U of restriction enzyme SfiI, and the resulting DNA fragments were recovered with a DNA CELL (Daiichi Kagaku). An approximately 48K Marcos DNA was obtained by the same method as in Example 10. The obtained Marcos DNA was verified by digestion with restriction enzyme KpnI present on pCHD2L but not on the expression unit, SfiI located on both ends of the expression unit and SacI on the expression unit. The obtained Marcos DNA was confirmed to have a plurality (about 20) of the Sfi fragments of PaSR-H1 introduced in the same direction per pCHD2L vector.

Transfection of the Marcos DNA into the CHO-K1 cells, selection and cloning of the cells in which the DNA had been introduced, and confirmation of expression of the AGPR L-H2 protein were conducted according to Example 10. As a result, clones were obtained for which expression of AGPR L-H2 protein was confirmed by Western blotting and expression of AGPR L-H2 protein was also confirmed by the indirect fluorescent antibody method in virtually 100% of the cells. The AGPR L-H2 protein was detected on the cell surfaces and cytoplasm.

### Example 12. Co-expression of AGPR H1 protein and AGPR L-H2 protein in cells of established cell line CHO-K1

The AGPR H1 gene and AGPR L-H2 gene was introduced into cells of the established animal cell line CHO-K1 to express both AGPR H1 protein and AGPR L-H2 protein. The method of expressing both the AGPR H1 protein and AGPR L-H2 protein in the CHO-K1 cells is described below.

The expression vector for introduction of the AGPR H1 gene and AGPR L-H2 gene into the CHO-K1 cells was constructed by the Marcos method as for AGPR H1 in Example 10. To prepare the H1 gene and L-H2 gene as the expression unit, DNA fragments obtained by digesting PaSR-H1 and PaSR-L-H2 with SfiI were collected with a DNA CELL (Daiichi Kagaku) and used, in the same manner as Examples 10 and 11. For the ligation, equivalent amounts of H1 and L-H2 SfiI fragments were used. An approximately 48K Marcos DNA was obtained by this method.

Confirmation with restriction enzymes was carried out in the same manner as Examples 10 and 11, fragments of molecular weight corresponding to SfiI of PaSR-H1 and PaSR-L-H2 were detected with SfiI, and DNA fragments were obtained at the specific restriction enzyme sites on the respective H1 and L-H2 genes. The obtained Marcos DNA was also used as a template for 25 cycles of PCR at 94°C for one minute, 55°C for one minute and 72°C for one minute, using primer F1/B9 specific to the AGPR H1 gene and primer 7/4 specific to the AGPR L-H2 gene, upon which an approximately 1.1 K amplified fragment corresponding to the desired domain of the H1 or L-H2 gene was detected.

The obtained Marcos DNA was confirmed to have a plurality (about 20) of the SfiI fragments of PaSR-H1 and Sfi fragments of PaSR-L-H2 introduced as expression units per pCHD2L vector.

Transfection of the Marcos DNA into the CHO-K1 cells, selection and cloning of the cells in which the DNA had been introduced, and confirmation of expression of the AGPR H1 and AGPR L-H2 protein were conducted according to Example 10.

As a result, clones were obtained for which co-expression of AGPR H1 and AGPR L-H2 protein was confirmed by Western blotting and co-expression of AGPR H1 and AGPR L-H2 protein was also confirmed by the indirect fluorescent antibody method in virtually 100% of the cells. The AGPR H1 and AGPR L-H2 proteins were both detected on the cell surfaces and cytoplasm.

### Example 13. Detection of anti-AGPR antibody in autoimmune hepatitis (AIH) patient serum

The AGPR protein-expressing CHO-K1 cells obtained in Examples 10, 11 and 12 were used to detect anti-AGPR antibodies in autoimmune hepatitis patient serum by the indirect fluorescent antibody method. The following procedure was followed.

The indirect fluorescent antibody method was conducted in the following manner. After culturing 1 × 10⁴ cells on a 8-well glass chamber slide for 3 days and washing with PBS(-), they were immobilized with acetone:methanol=1:1, and air-dried. As the primary antibody, AIH patient serum and control serum were diluted 100-fold with PBS(-) and incubated for 30 minutes at 37°C. After washing with PBS(-), anti-human IgG-fluorescein was used as the secondary antibody and incubation was continued at 37°C for 30 minutes. After further washing with PBS(-) and air drying followed by glycerol encapsulation, the cells were observed with a fluorescent microscope.

Of the 4 AIH patient serum specimens used as the primary antibody, with one specimen strong fluorescence was observed in the cytoplasm of CHO-K1 cells expressing both AGPR H1 and AGPR L-H2 protein. Only slight fluorescence was observed in the cytoplasm of CHO-K1 cells expressing AGPR H1 alone or AGPR L-H2 alone. No fluorescence was observed in the cytoplasm of wild CHO-K1 cells. Fluorescence was found in the nuclei of the cells in all 4 of the AIH patient serum specimens. However, in 3 of the specimens, no fluorescence was found in the cytoplasm of any of the cells. None of the cells in the 4 control serum specimens showed fluorescence in the cytoplasm or nucleus.

### Example 14. Assay of anti-AGPR antibody in patient serum by ELISA

The details regarding the human serum specimens were: 38 cases of autoimmune hepatitis (AIH), 30 cases of primary biliary cirrhosis (PBC), 10 cases of alcoholic liver disease (ALD), 2 cases of systemic lupus erythematosus (SLE), 16 cases of chronic hepatitis B (CH-B) and 19 healthy cases. The anti-AGPR antibodies in the sera were assayed by ELISA, according to the following method.

The recombinant AGPR proteins H1TM(-) and L-H2TM(-) were diluted with PBS(-) and dispensed at 1 µg/100 µl/well, and allowed to stand at room temperature for 2 hours for coating onto an ELISA immunomodule plate (Nunc, Maxisorp). After washing and removal of the antigen, 0.5% casein/PBS(-) was dispensed at 300 µl/well as a blocking buffer solution, and the mixture was allowed to stand at 4°C overnight. After removal of the blocking buffer solution, diluted samples of the human serum specimens were added at 100 µl/well prior for incubation at 37°C for 2 hours. The dilution was carried out using 0.05% casein/1% BSA/0.05% Tween20/PBS(-), and the degree of dilution was 1000-fold.

After washing and removal of the serum, peroxidase-labelled anti-human IgG polyclonal antibody (BioRad) was diluted with 0.05% casein/1% BSA/0.05% Tween20/PBS(-) to 10,000-fold for antigen H1TM(-) and to 5000-fold for antigen L-H2TM(-), and dispensed at 100 µl/well prior to incubation at 37°C for 2 hours. After washing and removal of this secondary antibody, a mixture of 2 mg/ml OPD (o-phenylenediamine)/0.059 M citric acid-0.039 M phosphate buffer solution (pH 3.4)/6 mM H₂O₂ was dispensed at 100 µl/well, and incubation was performed at room temperature for 15 minutes for coloration, and then after adding 10% sulfuric acid at 100 µl/well, the absorbance at O.D. 492 nm was measured with a microplate reader (CORONA). Washing was performed 4 times with 0.05% Tween20/PBS(-) for the washing/removal procedures in each reaction step.

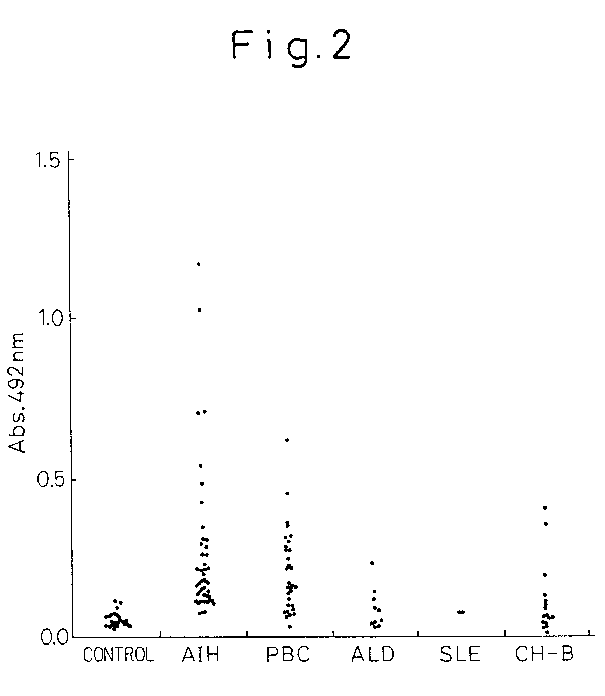

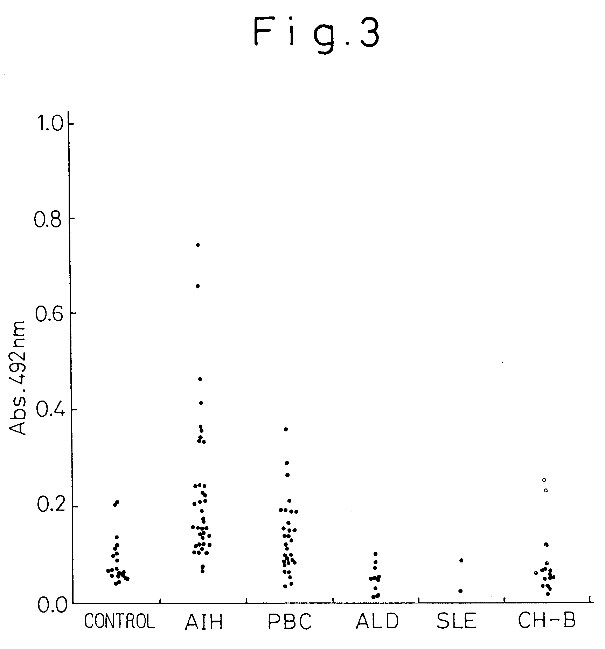

As shown by the graphs of values for the antigens according to case type, the AIH and PBC groups showed a distribution at higher values than the ALD, SLE, CH-B and normal groups (Figs. 2 and 3). This anti-AGPR antibody detection system distinguished the groups of AIH and PBC, which are autoimmune liver diseases, from the group of healthy persons, the group of ALD and SLE which are other autoimmune diseases, and the group of chronic hepatitis B.

SEQUENCE LISTING

(1) GENERAL INFORMATION:

(i) APPLICANT:
(A) NAME: Tonen Corporation
(B) STREET: 1-1, Hitotsubashi 1-chome, Chiyoda-ku
(C) CITY: Tokyo
(E) COUNTRY: Japan
(F) POSTAL CODE (ZIP): none

(ii) TITLE OF INVENTION: Asialoglycoprotein Receptor Derivatives and
Their Use

(iii) NUMBER OF SEQUENCES: 22

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96113349.3-1270

(2) INFORMATION FOR SEQ ID NO: 1:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 1:

CACTGAAGAA CCTGGGAATC AGAC 24

(2) INFORMATION FOR SEQ ID NO: 2:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 2:

AGCCCTATCA TGACCAAGGA G 21

(2) INFORMATION FOR SEQ ID NO: 3:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 3:

CAGGTCGAGG CATTGAAGA 19

(2) INFORMATION FOR SEQ ID NO: 4:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 4:

TTTCAAGCTC CTCACCTTCG G 21

(2) INFORMATION FOR SEQ ID NO: 5:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 5:

ATCATGGCCA AGGACTTTCA A 21

(2) INFORMATION FOR SEQ ID NO: 6:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 6:

ATGGGTTAGC CAGAGGTGTG CT 22

(2) INFORMATION FOR SEQ ID NO: 7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 7:

ATGGTCTGCT TCAGTCTGCT TGCC 24

(2) INFORMATION FOR SEQ ID NO: 8:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 8:

CTGTCTCAGT GTTTCTTCCT TTCC 24

(2) INFORMATION FOR SEQ ID NO: 9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 9:

CCCCAGTTCT CCTGGCTTTA AC 22

(2) INFORMATION FOR SEQ ID NO: 10:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 10:

GGCATCGTGA TCTGCTTTCA GG 22

(2) INFORMATION FOR SEQ ID NO: 11:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 11:

GAATTCGGAT CCCAAAACTC CCAG 24

(2) INFORMATION FOR SEQ ID NO: 12:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 12:

GTCGACTTAA AGGAGAGGTG GCTC 24

(2) INFORMATION FOR SEQ ID NO: 13:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 13:

CTCGAGATGA CCAAGGAGTA TCAAG 25

(2) INFORMATION FOR SEQ ID NO: 14:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 14:

GAATTCGAGG AGGCGAGGTC CGGA 24

(2) INFORMATION FOR SEQ ID NO: 15:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 15:

GAATTCGGGT CCCAAAGTGC ACAG 24

(2) INFORMATION FOR SEQ ID NO: 16:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 16:

GGATCCTTAT CAGGCCACCT CGCCGGT 27

(2) INFORMATION FOR SEQ ID NO: 17:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 17:

CTCGAGATGG CCAAGGACTT TCAA 24

(2) INFORMATION FOR SEQ ID NO: 18:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 18:

GAATTCACTG AAGCAGACCA TGGAG 25

(2) INFORMATION FOR SEQ ID NO: 19:

(i) SEQUENCE CHARACTERISTICS:
(A) LENGTH: 1277 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION:173..1045

(xi) SEQUENCE DESCRIPTION: SEQ ID NO: 19:

AGGGCCCTCC TATGGACCCT GCCCGCTCCC CTCCCATTGT CCACGGCTGT CCGCCCACCC 60

CCATTCTCCA AGCTTCAGCC CCCTCCTTAG TTCGGCATCT GCACAGCACT GAAGAACCTG 120

GGAATCAGAC CCTGAGACCC TGAGCAATCC CAGGTCCAGC GCCAGCCCTA TC ATG 175
Met
1

ACC AAG GAG TAT CAA GAC CTT CAG CAT CTG GAC AAT GAG GAG AGT GAC 223
Thr Lys Glu Tyr Gln Asp Leu Gln His Leu Asp Asn Glu Glu Ser Asp
            5                   10                  15

CAC CAT CAG CTC AGA AAA GGG CCA CCT CCT CCC CAG CCC CTC CTG CAG 271
His His Gln Leu Arg Lys Gly Pro Pro Pro Pro Gln Pro Leu Leu Gln
        20                  25                  30

CGT CTC TGC TCC GGA CCT CGC CTC CTC CTG CTC TCC CTG GGC CTC AGC 319
Arg Leu Cys Ser Gly Pro Arg Leu Leu Leu Leu Ser Leu Gly Leu Ser
    35                  40                  45

CTC CTG CTG CTT GTG GTT GTC TGT GTG ATC GGA TCC CAA AAC TCC CAG 367
Leu Leu Leu Leu Val Val Val Cys Val Ile Gly Ser Gln Asn Ser Gln
50                  55                  60                  65

CTG CAG GAG GAG CTG CGG GGC CTG AGA GAG ACG TTC AGC AAC TTC ACA 415
Leu Gln Glu Glu Leu Arg Gly Leu Arg Glu Thr Phe Ser Asn Phe Thr
                70                  75                  80

GCG AGC ACG GAG GCC CAG GTC AAG GGC TTG AGC ACC CAG GGA GGC AAT 463
Ala Ser Thr Glu Ala Gln Val Lys Gly Leu Ser Thr Gln Gly Gly Asn
            85                  90                  95

GTG GGA AGA AAG ATG AAG TCG CTA GAG TCC CAG CTG GAG AAA CAG CAG 511
Val Gly Arg Lys Met Lys Ser Leu Glu Ser Gln Leu Glu Lys Gln Gln
        100                 105                 110

AAG GAC CTG AGT GAA GAT CAC TCC AGC CTG CTG CTC CAC GTG AAG CAG 559
Lys Asp Leu Ser Glu Asp His Ser Ser Leu Leu Leu His Val Lys Gln
    115                 120                 125

TTC GTG TCT GAC CTG CGG AGC CTG AGC TGT CAG ATG GCG GCG CTC CAG 607
Phe Val Ser Asp Leu Arg Ser Leu Ser Cys Gln Met Ala Ala Leu Gln
130                 135                 140                 145

GGC AAT GGC TCA GAA AGG ACC TGC TGC CCG GTC AAC TGG GTG GAG CAC 655
Gly Asn Gly Ser Glu Arg Thr Cys Cys Pro Val Asn Trp Val Glu His
                150                 155                 160

GAG CGC AGC TGC TAC TGG TTC TCT CGC TCC GGG AAG GCC TGG GCT GAC 703
Glu Arg Ser Cys Tyr Trp Phe Ser Arg Ser Gly Lys Ala Trp Ala Asp
            165                 170                 175

GCC GAC AAC TAC TGC CGG CTG GAG GAC GCG CAC CTG GTG GTG GTC ACG 751
Ala Asp Asn Tyr Cys Arg Leu Glu Asp Ala His Leu Val Val Val Thr
        180                 185                 190

TCC TGG GAG GAG CAG AAA TTT GTC CAG CAC CAC ATA GGC CCT GTG AAC 799
Ser Trp Glu Glu Gln Lys Phe Val Gln His His Ile Gly Pro Val Asn
    195                 200                 205

ACC TGG ATG GGC CTC CAC GAC CAA AAC GGG CCC TGG AAG TGG GTG GAC 847
Thr Trp Met Gly Leu His Asp Gln Asn Gly Pro Trp Lys Trp Val Asp
210                 215                 220                 225

GGG ACG GAC TAC GAG ACG GGC TTC AAG AAC TGG AGG CCG GAG CAG CCG 895
Gly Thr Asp Tyr Glu Thr Gly Phe Lys Asn Trp Arg Pro Glu Gln Pro
                230                 235                 240

GAC GAC TGG TAC GGC CAC GGG CTC GGA GGA GGC GAG GAC TGT GCC CAC 943
Asp Asp Trp Tyr Gly His Gly Leu Gly Gly Gly Glu Asp Cys Ala His
            245                 250                 255

TTC ACC GAC GAC GGC CGC TGG AAC GAC GAC GTC TGC CAG AGG CCC TAC 991
Phe Thr Asp Asp Gly Arg Trp Asn Asp Asp Val Cys Gln Arg Pro Tyr
        260                 265                 270

CGC TGG GTC TGC GAG ACA GAG CTG GAC AAG GCC AGC CAG GAG CCA CCT 1039
Arg Trp Val Cys Glu Thr Glu Leu Asp Lys Ala Ser Gln Glu Pro Pro
    275                 280                 285

CTC CTT TAATTTATTT CTTCAATGCC TCGACCTGCC GCAGGGGTCC GGGATTGGGA 1095
Leu Leu
290

ATCCGCCCAT CTGGGGCCTC TTCTGCTTTC TCGGGAATTT TCATCTAGGA TTTTAAGGGA 1155

AGGGGAAGGA TAGGGTGATG TTCCGAAGGT GAGGAGCTTG AAACCCGTGG CGCTTTCTGC 1215

AGTTCACAAT GATAACCTGC AAACTGCAGA AAGCGCCACG GGTTTCAAGC TCCTCACCTT 1275

CG 1277

(2) INFORMATION FOR SEQ ID NO: 20:

(i) SEQUENCE CHARACTERISTICS:
(A) LENGTH: 291 amino acids
(B) TYPE: amino acid
(D) TOPOLOGY: linear

(ii) MOLECULE TYPE: protein
(xi) SEQUENCE DESCRIPTION: SEQ ID NO: 20:

Met Thr Lys Glu Tyr Gln Asp Leu Gln His Leu Asp Asn Glu Glu Ser
1               5                   10                  15

Asp His His Gln Leu Arg Lys Gly Pro Pro Pro Pro Gln Pro Leu Leu
            20                  25                  30

Gln Arg Leu Cys Ser Gly Pro Arg Leu Leu Leu Leu Ser Leu Gly Leu
        35                  40                  45

Ser Leu Leu Leu Leu Val Val Val Cys Val Ile Gly Ser Gln Asn Ser
    50                  55                  60

Gln Leu Gln Glu Glu Leu Arg Gly Leu Arg Glu Thr Phe Ser Asn Phe
65                  70                  75                  80

Thr Ala Ser Thr Glu Ala Gln Val Lys Gly Leu Ser Thr Gln Gly Gly
                85                  90                  95

Asn Val Gly Arg Lys Met Lys Ser Leu Glu Ser Gln Leu Glu Lys Gln
            100                 105                 110

Gln Lys Asp Leu Ser Glu Asp His Ser Ser Leu Leu Leu His Val Lys
        115                 120                 125

Gln Phe Val Ser Asp Leu Arg Ser Leu Ser Cys Gln Met Ala Ala Leu
    130                 135                 140

Gln Gly Asn Gly Ser Glu Arg Thr Cys Cys Pro Val Asn Trp Val Glu
145                 150                 155                 160

His Glu Arg Ser Cys Tyr Trp Phe Ser Arg Ser Gly Lys Ala Trp Ala
                165                 170                 175

Asp Ala Asp Asn Tyr Cys Arg Leu Glu Asp Ala His Leu Val Val Val
            180                 185                 190

Thr Ser Trp Glu Glu Gln Lys Phe Val Gln His His Ile Gly Pro Val
        195                 200                 205

Asn Thr Trp Met Gly Leu His Asp Gln Asn Gly Pro Trp Lys Trp Val
    210                 215                 220

Asp Gly Thr Asp Tyr Glu Thr Gly Phe Lys Asn Trp Arg Pro Glu Gln
225                 230                 235                 240

Pro Asp Asp Trp Tyr Gly His Gly Leu Gly Gly Gly Glu Asp Cys Ala
                245                 250                 255

His Phe Thr Asp Asp Gly Arg Trp Asn Asp Asp Val Cys Gln Arg Pro
            260                 265                 270

Tyr Arg Trp Val Cys Glu Thr Glu Leu Asp Lys Ala Ser Gln Glu Pro
        275                 280                 285

Pro Leu Leu
    290

GTG GAG CAC CAA GGC AGC TGC TAC TGG TTC TCT CAC TCC GGG AAG GCC     709
Val Glu His Gln Gly Ser Cys Tyr Trp Phe Ser His Ser Gly Lys Ala
    450                 455                 460

TGG GCT GAG GCG GAG AAG TAC TGC CAG CTG GAG AAC GCA CAC CTG GTG     757
Trp Ala Glu Ala Glu Lys Tyr Cys Gln Leu Glu Asn Ala His Leu Val
465                 470                 475                 480

GTC ATC AAC TCC TGG GAG GAG CAG AAA TTC ATT GTA CAA CAC ACG AAC     805
Val Ile Asn Ser Trp Glu Glu Gln Lys Phe Ile Val Gln His Thr Asn
                485                 490                 495

CCC TTC AAT ACC TGG ATA GGT CTC ACG GAC AGT GAT GGC TCT TGG AAA     853
Pro Phe Asn Thr Trp Ile Gly Leu Thr Asp Ser Asp Gly Ser Trp Lys
            500                 505                 510

TGG GTG GAT GGC ACA GAC TAT AGG CAC AAC TAC AAG AAC TGG GCT GTC     901
Trp Val Asp Gly Thr Asp Tyr Arg His Asn Tyr Lys Asn Trp Ala Val
        515                 520                 525

ACT CAG CCA GAT AAT TGG CAC GGG CAC GAG CTG GGT GGA AGT GAA GAC     949
Thr Gln Pro Asp Asn Trp His Gly His Glu Leu Gly Gly Ser Glu Asp
    530                 535                 540

TGT GTT GAA GTC CAG CCG GAT GGC CGC TGG AAC GAT GAC TTC TGC CTG     997
Cys Val Glu Val Gln Pro Asp Gly Arg Trp Asn Asp Asp Phe Cys Leu
545                 550                 555                 560

CAG GTG TAC CGC TGG GTG TGT GAG AAA AGG CGG AAT GCC ACC GGC GAG     1045
Gln Val Tyr Arg Trp Val Cys Glu Lys Arg Arg Asn Ala Thr Gly Glu
                565                 570                 575

GTG GCC TGACCCCAGC ACACCTCTGG CTAACCCATA CCCCACACCT GCCCAGCTCT     1101
Val Ala

GGCTTCTCTG TTGAGGATTT TGAGGAAAGG AAGAAACACT GAGACAGGGG TATGGGGAAG     1161

AGCTGAGCAA AGAGAGAAAG GAGGTAGTTT AAGAGTCCCT GACCCTGGAG GACTGAGATC     1221

CCACCTCCTT CTGTAATTCA TTGTAATTAT TATAATCGTC AGCCTCTTCA ATGGCGTAGG     1281

AAAGAAGAAA CAAATGCTT     1300

(2) INFORMATION FOR SEQ ID NO: 22:

(i) SEQUENCE CHARACTERISTICS:
(A) LENGTH: 287 amino acids
(B) TYPE: amino acid
(D) TOPOLOGY: linear

(ii) MOLECULE TYPE: protein
(xi) SEQUENCE DESCRIPTION: SEQ ID NO: 22:

Met Ala Lys Asp Phe Gln Asp Ile Gln Gln Leu Ser Ser Glu Glu Asn
  1               5                  10                  15

Asp His Pro Phe His Gln Gly Pro Pro Pro Ala Gln Pro Leu Ala Gln
             20                  25                  30

Arg Leu Cys Ser Met Val Cys Phe Ser Leu Leu Ala Leu Ser Phe Asn
         35                  40                  45

Ile Leu Leu Leu Val Val Ile Cys Val Thr Gly Ser Gln Ser Ala Gln
     50                  55                  60

(2) INFORMATION FOR SEQ ID NO: 21:

(i) SEQUENCE CHARACTERISTICS:
(A) LENGTH: 1300 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION:191..1051

(xi) SEQUENCE DESCRIPTION: SEQ ID NO: 21:

CTGCACTCTC CTCTCCCCTG TGAGCTCCAC CTGCCCCAGT TCTCCTGGCT TTAACCCCTC 60

CTTGGCCAAG GCCAGGGTTG CCTGCGGGAG CCAGGCGTCC GCTCTCCACA CCTTTCACAG 120

CCCCAGCCCT CAGAGCAACC TCAGCCCAGC CCAGCCCAGC TCCAGCTCCA GCTCCAGCCC 180

GGGCCCCATC ATG GCC AAG GAC TTT CAA GAT ATC CAG CAG CTG AGC TCG 229
Met Ala Lys Asp Phe Gln Asp Ile Gln Gln Leu Ser Ser
295 300

GAG GAA AAT GAC CAT CCT TTC CAT CAA GGG CCA CCT CCT GCC CAG CCC 277
Glu Glu Asn Asp His Pro Phe His Gln Gly Pro Pro Pro Ala Gln Pro
305 310 315 320

CTG GCA CAG CGT CTC TGC TCC ATG GTC TGC TTC AGT CTG CTT GCC CTG 325
Leu Ala Gln Arg Leu Cys Ser Met Val Cys Phe Ser Leu Leu Ala Leu
325 330 335

AGC TTC AAC ATC CTG CTG CTG GTG GTC ATC TGT GTG ACT GGG TCC CAA 373
Ser Phe Asn Ile Leu Leu Leu Val Val Ile Cys Val Thr Gly Ser Gln
340 345 350

AGT GCA CAG CTG CAA GCC GAG CTG CGG AGC CTG AAG GAA GCT TTC AGC 421
Ser Ala Gln Leu Gln Ala Glu Leu Arg Ser Leu Lys Glu Ala Phe Ser
355 360 365

AAC TTC TCC TCG AGC ACC CTG ACG GAG GTC CAG GCA ATC AGC ACC CAC 469
Asn Phe Ser Ser Ser Thr Leu Thr Glu Val Gln Ala Ile Ser Thr His
370 375 380

GGA GGC AGC GTG GGT GAC AAG ATC ACA TCC CTA GGA GCC AAG CTG GAG 517
Gly Gly Ser Val Gly Asp Lys Ile Thr Ser Leu Gly Ala Lys Leu Glu
385 390 395 400

AAA CAG CAG CAG GAC CTG AAA GCA GAT CAC GAT GCC CTG CTC TTC CAT 565
Lys Gln Gln Gln Asp Leu Lys Ala Asp His Asp Ala Leu Leu Phe His
405 410 415

CTG AAG CAC TTC CCC GTG GAC CTG CGC TTC GTG GCC TGC CAG ATG GAG 613
Leu Lys His Phe Pro Val Asp Leu Arg Phe Val Ala Cys Gln Met Glu
420 425 430

CTC CTC CAC AGC AAC GGC TCC CAA AGG ACC TGC TGC CCC GTC AAC TGG 661
Leu Leu His Ser Asn Gly Ser Gln Arg Thr Cys Cys Pro Val Asn Trp
435 440 445

Leu Gln Ala Glu Leu Arg Ser Leu Lys Glu Ala Phe Ser Asn Phe Ser
65                  70                  75                  80

Ser Ser Thr Leu Thr Glu Val Gln Ala Ile Ser Thr His Gly Gly Ser
                85                  90                  95

Val Gly Asp Lys Ile Thr Ser Leu Gly Ala Lys Leu Glu Lys Gln Gln
            100                 105                 110

Gln Asp Leu Lys Ala Asp His Asp Ala Leu Leu Phe His Leu Lys His
        115                 120                 125

Phe Pro Val Asp Leu Arg Phe Val Ala Cys Gln Met Glu Leu Leu His
    130                 135                 140

Ser Asn Gly Ser Gln Arg Thr Cys Cys Pro Val Asn Trp Val Glu His
145                 150                 155                 160

Gln Gly Ser Cys Tyr Trp Phe Ser His Ser Gly Lys Ala Trp Ala Glu
                165                 170                 175

Ala Glu Lys Tyr Cys Gln Leu Glu Asn Ala His Leu Val Val Ile Asn
            180                 185                 190

Ser Trp Glu Glu Gln Lys Phe Ile Val Gln His Thr Asn Pro Phe Asn
        195                 200                 205

Thr Trp Ile Gly Leu Thr Asp Ser Asp Gly Ser Trp Lys Trp Val Asp
    210                 215                 220

Gly Thr Asp Tyr Arg His Asn Tyr Lys Asn Trp Ala Val Thr Gln Pro
225                 230                 235                 240

Asp Asn Trp His Gly His Glu Leu Gly Gly Ser Glu Asp Cys Val Glu
                245                 250                 255

Val Gln Pro Asp Gly Arg Trp Asn Asp Asp Phe Cys Leu Gln Val Tyr
            260                 265                 270

Arg Trp Val Cys Glu Lys Arg Arg Asn Ala Thr Gly Glu Val Ala
        275                 280                 285

SEQUENCE LISTING
SEQ ID NO: 1
Sequence Length: 24
Sequence Type: nucleic acid
Strandedness: single
Topology: linear
Molecular Type: synthetic DNA
Sequence
CACTGAAGAA CCTGGGAATC AGAC 24
SEQ ID NO: 2
Sequence Length: 21
Sequence Type: nucleic acid
Strandedness: single
Topology: linear
Molecular Type: synthetic DNA
Sequence
AGCCCTATCA TGACCAAGGA G 21
SEQ ID NO: 3
Sequence Length: 19
Sequence Type: nucleic acid
Strandedness: single
Topology: linear
Molecular Type: synthetic DNA
Sequence
CAGGTCGAGG CATTGAAGA 19
SEQ ID NO: 4
Sequence Length: 21
Sequence Type: nucleic acid
Strandedness: single
Topology: linear
Molecular Type: synthetic DNA
Sequence
TTTCAAGCTC CTCACCTTCG G 21
SEQ ID NO: 5
Sequence Length: 21
Sequence Type: nucleic acid
Strandedness: single

Topology: linear
Molecular Type: synthetic DNA
Sequence
ATCATGGCCA AGGACTTTCA A 21
SEQ ID NO: 6
Sequence Length: 22
Sequence Type: nucleic acid
Strandedness: single
Topology: linear
Molecular Type: synthetic DNA
Sequence
ATGGGTTAGC CAGAGGTGTG CT 22
SEQ ID NO: 7
Sequence Length: 24
Sequence Type: nucleic acid
Strandedness: single
Topology: linear
Molecular Type: synthetic DNA
Sequence
ATGGTCTGCT TCAGTCTGCT TGCC 24
SEQ ID NO: 8
Sequence Length: 24
Sequence Type: nucleic acid
Strandedness: single
Topology: linear
Molecular Type: synthetic DNA
CTGTCTCAGT GTTTCTTCCT TTCC 24
SEQ ID NO: 9
Sequence Length: 22
Sequence Type: nucleic acid
Strandedness: single
Topology: linear
Molecular Type: synthetic DNA
Sequence
CCCCAGTTCT CCTGGCTTTA AC 22
SEQ ID NO: 10
Sequence Length: 22

Sequence Type: nucleic acid
Strandedness: single
Topology: linear
Molecular Type: synthetic DNA
Sequence
GGCATCGTGA TCTGCTTTCA GG 22
SEQ ID NO: 11
Sequence Length: 24
Sequence Type: nucleic acid
Strandedness: single
Topology: linear
Molecular Type: synthetic DNA
Sequence
GAATTCGGAT CCCAAAACTC CCAG 24
SEQ ID NO: 12
Sequence Length: 24
Sequence Type: nucleic acid
Strandedness: single
Topology: linear
Molecular Type: synthetic DNA
Sequence
GTCGACTTAA AGGAGAGGTG GCTC 24
SEQ ID NO: 13
Sequence Length: 25
Sequence Type: nucleic acid
Strandedness: single
Topology: linear
Molecular Type: synthetic DNA
Sequence
CTCGAGATGA CCAAGGAGTA TCAAG 25
SEQ ID NO: 14
Sequence Length: 24
Sequence Type: nucleic acid
Strandedness: single
Topology: linear
Molecular Type: synthetic DNA
Sequence

GAATTCGAGG AGGCGAGGTC CGGA 24
SEQ ID NO: 15
Sequence Length: 24
Sequence Type: nucleic acid
Strandedness: single
Topology: linear
Molecular Type: synthetic DNA
Sequence
GAATTCGGGT CCCAAAGTGC ACAG 24
SEQ ID NO: 16
Sequence Length: 27
Sequence Type: nucleic acid
Strandedness: single
Topology: linear
Molecular Type: synthetic DNA
Sequence
GGATCCTTAT CAGGCCACCT CGCCGGT 27
SEQ ID NO: 17
Sequence Length: 24
Sequence Type: nucleic acid
Strandedness: single
Topology: linear
Molecular Type: synthetic DNA
Sequence
CTCGAGATGG CCAAGGACTT TCAA 24
SEQ ID NO: 18
Sequence Length: 25
Sequence Type: nucleic acid
Strandedness: single
Topology: linear
Molecular Type: synthetic DNA
Sequence
GAATTCACTG AAGCAGACCA TGGAG 25
SEQ ID NO: 19
Sequence Length: 1277
Sequence Type: nucleic acid
Strandedness: double

Topology: linear
Molecular Type: cDNA
Sequence
AGGGCCCTCC TATGGACCCT GCCCGCTCCC CTCCCATTGT CCACGGCTGT CCGCCCACCC 60
CCATTCTCCA AGCTTCAGCC CCCTCCTTAG TTCGGCATCT GCACAGCACT GAAGAACCTG 120
GGAATCAGAC CCTGAGACCC TGAGCAATCC CAGGTCCAGC GCCAGCCCTA TC ATG ACC 178
Met Thr
AAG GAG TAT CAA GAC CTT CAG CAT CTG GAC AAT GAG GAG AGT GAC CAC 226
Lys Glu Tyr Gln Asp Leu Gln His Leu Asp Asn Glu Glu Ser Asp His
        5                   10                  15
CAT CAG CTC AGA AAA GGG CCA CCT CCT CCC CAG CCC CTC CTG CAG CGT 274
His Gln Leu Arg Lys Gly Pro Pro Pro Pro Gln Pro Leu Leu Gln Arg
    20                  25                  30
CTC TGC TCC GGA CCT CGC CTC CTC CTG CTC TCC CTG GGC CTC AGC CTC 322
Leu Cys Ser Gly Pro Arg Leu Leu Leu Leu Ser Leu Gly Leu Ser Leu
35                  40                  45                  50
CTG CTG CTT GTG GTT GTC TGT GTG ATC GGA TCC CAA AAC TCC CAG CTG 370
Leu Leu Leu Val Val Val Cys Val Ile Gly Ser Gln Asn Ser Gln Leu
                55                  60                  65
CAG GAG GAG CTG CGG GGC CTG AGA GAG ACG TTC AGC AAC TTC ACA GCG 418
Gln Glu Glu Leu Arg Gly Leu Arg Glu Thr Phe Ser Asn Phe Thr Ala
            70                  75                  80
AGC ACG GAG GCC CAG GTC AAG GGC TTG AGC ACC CAG GGA GGC AAT GTG 466
Ser Thr Glu Ala Gln Val Lys Gly Leu Ser Thr Gln Gly Gly Asn Val
        85                  90                  95
GGA AGA AAG ATG AAG TCG CTA GAG TCC CAG CTG GAG AAA CAG CAG AAG 514
Gly Arg Lys Met Lys Ser Leu Glu Ser Gln Leu Glu Lys Gln Gln Lys
    100                 105                 110
GAC CTG AGT GAA GAT CAC TCC AGC CTG CTG CTC CAC GTG AAG CAG TTC 562
Asp Leu Ser Glu Asp His Ser Ser Leu Leu Leu His Val Lys Gln Phe
115                 120                 125                 130
GTG TCT GAC CTG CGG AGC CTG AGC TGT CAG ATG GCG GCG CTC CAG GGC 610
Val Ser Asp Leu Arg Ser Leu Ser Cys Gln Met Ala Ala Leu Gln Gly
                135                 140                 145
AAT GGC TCA GAA AGG ACC TGC TGC CCG GTC AAC TGG GTG GAG CAC GAG 658
Asn Gly Ser Glu Arg Thr Cys Cys Pro Val Asn Trp Val Glu His Glu
            150                 155                 160
CGC AGC TGC TAC TGG TTC TCT CGC TCC GGG AAG GCC TGG GCT GAC GCC 706
Arg Ser Cys Tyr Trp Phe Ser Arg Ser Gly Lys Ala Trp Ala Asp Ala
        165                 170                 175
GAC AAC TAC TGC CGG CTG GAG GAC GCG CAC CTG GTG GTG GTC ACG TCC 754
Asp Asn Tyr Cys Arg Leu Glu Asp Ala His Leu Val Val Val Thr Ser
    180                 185                 190

TGG GAG GAG CAG AAA TTT GTC CAG CAC CAC ATA GGC CCT GTG AAC ACC 802
Trp Glu Glu Gln Lys Phe Val Gln His His Ile Gly Pro Val Asn Thr
195                 200                 205                 210
TGG ATG GGC CTC CAC GAC CAA AAC GGG CCC TGG AAG TGG GTG GAC GGG 850
Trp Met Gly Leu His Asp Gln Asn Gly Pro Trp Lys Trp Val Asp Gly
                215                 220                 225
ACG GAC TAC GAG ACG GGC TTC AAG AAC TGG AGG CCG GAG CAG CCG GAC 898
Thr Asp Tyr Glu Thr Gly Phe Lys Asn Trp Arg Pro Glu Gln Pro Asp
            230                 235                 240
GAC TGG TAC GGC CAC GGG CTC GGA GGA GGC GAG GAC TGT GCC CAC TTC 946
Asp Trp Tyr Gly His Gly Leu Gly Gly Gly Glu Asp Cys Ala His Phe
        245                 250                 255
ACC GAC GAC GGC CGC TGG AAC GAC GAC GTC TGC CAG AGG CCC TAC CGC 994
Thr Asp Asp Gly Arg Trp Asn Asp Asp Val Cys Gln Arg Pro Tyr Arg
    260                 265                 270
TGG GTC TGC GAG ACA GAG CTG GAC AAG GCC AGC CAG GAG CCA CCT CTC 1042
Trp Val Cys Glu Thr Glu Leu Asp Lys Ala Ser Gln Glu Pro Pro Leu
275                 280                 285                 290
CTT TAATTTATTT CTTCAATGCC TCGACCTGCC GCAGGGGTCC GGGATTGGGA 1095
Leu
ATCCGCCCAT CTGGGGCCTC TTCTGCTTTC TCGGGAATTT TCATCTAGGA TTTTAAGGGA 1155
AGGGGAAGGA TAGGGTGATG TTCCGAAGGT GAGGAGCTTG AAACCCGTGG CGCTTTCTGC 1215
AGTTCACAAT GATAACCTGC AAACTGCAGA AAGCGCCACG GGTTTCAAGC TCCTCACCTT 1275
CG 1277
SEQ ID NO: 20
Sequence Length: 1300
Sequence Type: nucleic acid
Strandedness: double
Topology: linear
Molecular Type: cDNA
Sequence
CTGCACTCTC CTCTCCCCTG TGAGCTCCAC CTGCCCCAGT TCTCCTGGCT TTAACCCCTC 60
CTTGGCCAAG GCCAGGGTTG CCTGCGGGAG CCAGGCGTCC GCTCTCCACA CCTTTCACAG 120
CCCCAGCCCT CAGAGCAACC TCAGCCCAGC CCAGCCCAGC TCCAGCTCCA GCTCCAGCCC 180
GGGCCCCATC ATG GCC AAG GAC TTT CAA GAT ATC CAG CAG CTG AGC TCG 229
Met Ala Lys Asp Phe Gln Asp Ile Gln Gln Leu Ser Ser
                5                   10
GAG GAA AAT GAC CAT CCT TTC CAT CAA GGG CCA CCT CCT GCC CAG CCC 277
Glu Glu Asn Asp His Pro Phe His Gln Gly Pro Pro Pro Ala Gln Pro
    15                  20                  25

CTG GCA CAG CGT CTC TGC TCC ATG GTC TGC TTC AGT CTG CTT GCC CTG 325
Leu Ala Gln Arg Leu Cys Ser Met Val Cys Phe Ser Leu Leu Ala Leu
30                  35                  40                  45
AGC TTC AAC ATC CTG CTG CTG GTG GTC ATC TGT GTG ACT GGG TCC CAA 373
Ser Phe Asn Ile Leu Leu Leu Val Val Ile Cys Val Thr Gly Ser Gln
                50                  55                  60
AGT GCA CAG CTG CAA GCC GAG CTG CGG AGC CTG AAG GAA GCT TTC AGC 421
Ser Ala Gln Leu Gln Ala Glu Leu Arg Ser Leu Lys Glu Ala Phe Ser
            65                  70                  75
AAC TTC TCC TCG AGC ACC CTG ACG GAG GTC CAG GCA ATC AGC ACC CAC 469
Asn Phe Ser Ser Ser Thr Leu Thr Glu Val Gln Ala Ile Ser Thr His
        80                  85                  90
GGA GGC AGC GTG GGT GAC AAG ATC ACA TCC CTA GGA GCC AAG CTG GAG 517
Gly Gly Ser Val Gly Asp Lys Ile Thr Ser Leu Gly Ala Lys Leu Glu
    95                  100                 105
AAA CAG CAG CAG GAC CTG AAA GCA GAT CAC GAT GCC CTG CTC TTC CAT 565
Lys Gln Gln Gln Asp Leu Lys Ala Asp His Asp Ala Leu Leu Phe His
110                 115                 120                 125
CTG AAG CAC TTC CCC GTG GAC CTG CGC TTC GTG GCC TGC CAG ATG GAG 613
Leu Lys His Phe Pro Val Asp Leu Arg Phe Val Ala Cys Gln Met Glu
                130                 135                 140
CTC CTC CAC AGC AAC GGC TCC CAA AGG ACC TGC TGC CCC GTC AAC TGG 661
Leu Leu His Ser Asn Gly Ser Gln Arg Thr Cys Cys Pro Val Asn Trp
            145                 150                 155
GTG GAG CAC CAA GGC AGC TGC TAC TGG TTC TCT CAC TCC GGG AAG GCC 709
Val Glu His Gln Gly Ser Cys Tyr Trp Phe Ser His Ser Gly Lys Ala
        160                 165                 170
TGG GCT GAG GCG GAG AAG TAC TGC CAG CTG GAG AAC GCA CAC CTG GTG 757
Trp Ala Glu Ala Glu Lys Tyr Cys Gln Leu Glu Asn Ala His Leu Val
    175                 180                 185
GTC ATC AAC TCC TGG GAG GAG CAG AAA TTC ATT GTA CAA CAC ACG AAC 805
Val Ile Asn Ser Trp Glu Glu Gln Lys Phe Ile Val Gln His Thr Asn
190                 195                 200                 205
CCC TTC AAT ACC TGG ATA GGT CTC ACG GAC AGT GAT GGC TCT TGG AAA 853
Pro Phe Asn Thr Trp Ile Gly Leu Thr Asp Ser Asp Gly Ser Trp Lys
                210                 215                 220
TGG GTG GAT GGC ACA GAC TAT AGG CAC AAC TAC AAG AAC TGG GCT GTC 901
Trp Val Asp Gly Thr Asp Tyr Arg His Asn Tyr Lys Asn Trp Ala Val
            225                 230                 235
ACT CAG CCA GAT AAT TGG CAC GGG CAC GAG CTG GGT GGA AGT GAA GAC 949
Thr Gln Pro Asp Asn Trp His Gly His Glu Leu Gly Gly Ser Glu Asp
        240                 245                 250

TGT GTT GAA GTC CAG CCG GAT GGC CGC TGG AAC GAT GAC TTC TGC CTG 997
Cys Val Glu Val Gln Pro Asp Gly Arg Trp Asn Asp Asp Phe Cys Leu
    255                 260                 265
CAG GTG TAC CGC TGG GTG TGT GAG AAA AGG CGG AAT GCC ACC GGC GAG 1045
Gln Val Tyr Arg Trp Val Cys Glu Lys Arg Arg Asn Ala Thr Gly Glu
270                 275                 280                 285
GTG GCC TGACCCCAGC ACACCTCTGG CTAACCCATA CCCCACACCT GCCCAGCTCT 1101
Val Ala
GGCTTCTCTG TTGAGGATTT TGAGGAAAGG AAGAAACACT GAGACAGGGG TATGGGGAAG 1161
AGCTGAGCAA AGAGAGAAAG GAGGTAGTTT AAGAGTCCCT GACCCTGGAG GACTGAGATC 1221
CCACCTCCTT CTGTAATTCA TTGTAATTAT TATAATCGTC AGCCTCTTCA ATGGCGTAGG 1281
AAAGAAGAAA CAAATGCTT 1300

## Claims

1. An asialoglycoprotein receptor H1 (AGPR H1) derivative comprising the extracellular domain of AGPR H1.

2. An asialoglycoprotein receptor L-H2 (AGPR L-H2) derivative comprising the extracellular domain of AGPR L-H2.

3. An AGPR H1 derivative comprising the cytoplasmic domain and extracellular domain of AGPR H1.

4. An AGPR L-H2 derivative comprising the cytoplasmic domain and extracellular domain of AGPR L-H2.

5. A process for production of an AGPR derivative according to any one of claims 1 to 4, comprising the steps of:
(1) culturing host cells transformed with an expression vector comprising DNA coding for the polypeptide amino acid sequence of said derivative, and
(2) recovering the cultured product or further collecting said AGPR derivative from the culture.

6. A process for production of an AGPR H1 or AGPR L-H2 comprising the steps of:
(1) culturing host cells transformed with an expression vector comprising DNA coding for the polypeptide amino acid sequence of said AGPR, and
(2) recovering the cultured product or further recovering said AGPR from the cultured product.

7. A process for simultaneously producing AGPR H1 and AGPR L-H2, comprising the steps of:
(1) culturing host cells transformed with an expression vector comprising DNA coding for the polypeptide amino acid sequence of said AGPR H1 and DNA coding for the polypeptide amino acid sequence of said AGPR L-H2, and
(2) recovering the cultured product or further recovering said AGPR H1 and AGPR L-H2 from the cultured product.

8. A process according to any one of claims 5 to 7, wherein said host cells are bacterial cells or animal cells.

9. A process according to claim 8 wherein said bacterial cells are *E. coli* cells.

10. A process according to claim 8 wherein said animal cells are mammalian cells.

11. A method of detecting or measuring an antibody against AGPR (anti-AGPR antibody), comprising the steps of:
(1) contacting a test sample to be measured for the presence or amount of the anti-AGPR antibody with at least one type of AGPR derivative according to any of claims 1 to 4 or AGPR produced by a method according to claim 6 or 7, and
(2) detecting whether an antigen/antibody reaction occurs or measuring its amount.

12. A method according to claim 11 wherein the method of measurement is the Enzyme-Linked Immunosorbent Assay (ELISA), Western blotting, the indirect fluorescent antibody method, immunoprecipitation, radioimmunoassay (RIA), immunodiffusion or immunoelectrophoresis.

13. A reagent for measuring anti-AGPR antibody which comprises at least one type of AGPR derivative according to any of claims 1 to 4 or AGPR obtained by a method according to claim 6 or 7.

14. A kit for measuring anti-AGPR antibody which comprises at least one type of AGPR derivative according to any of claims 1 to 4 or AGPR obtained by a method according to claim 6 or 7, and standard anti-AGPR antibody.
